(19) Europäisches Patentamt
European Patent Office
Office européen des brevets

(11) Numéro de publication : **0 524 846 A1**

(12) # DEMANDE DE BREVET EUROPEEN

(21) Numéro de dépôt : **92401769.2**

(51) Int. Cl.[5] : **C07D 401/06,** A61K 31/45

(22) Date de dépôt : **24.06.92**

(30) Priorité : **27.06.91 FR 9107934**
**27.06.91 FR 9107936**
**13.04.92 FR 9204507**

(43) Date de publication de la demande :
**27.01.93 Bulletin 93/04**

(84) Etats contractants désignés :
**AT BE CH DE DK ES FR GB GR IT LI LU MC NL PT SE**

(71) Demandeur : **SYNTHELABO**
**22, Avenue Galilée**
**F-92350 Le Plessis Robinson (FR)**

(72) Inventeur : **Frost, Jonathan**
**23, route de Montjean**
**F-91320 Wissous (FR)**
Inventeur : **Lardenois, Patrick**
**18, rue Varengue**
**F-92340 Bourg la Reine (FR)**
Inventeur : **Renones, Maria-Carmen**
**8, boulevard d'Ormesson**
**F-95880 Enghine-les-Bains (FR)**

(74) Mandataire : **Ludwig, Jacques**
**SYNTHELABO, Service Brevets, 22, avenue Galilée, B.P. 72**
**F-92352 Le Plessis Robinson Cédex (FR)**

(54) **Dérivés de 2-(pipéridin-1-y1) éthanol, leur préparation et leur application en thérapeutique.**

(57) Composé, sous forme d'isomère optique pur ou de mélange de deux énantiomères, répondant à la formule générale (I)

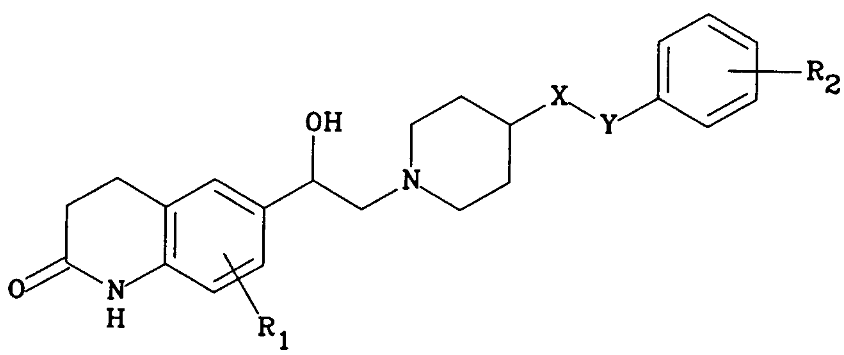

(I)

dans laquelle $R_1$ représente un atome d'hydrogène ou d'halogène ou un groupe méthyle, $R_2$ représente un ou deux substituants choisis parmi les atomes d'hydrogène et d'halogènes et les groupes $(C_{1-4})$alkyle, hydroxy, $(C_{1-4})$alcoxy, trifluorométhyle, acétylamino et méthylsulfonylamino, et soit X représente un groupe $CH_2$ et Y représente un groupe $CH_2$, $(CH_2)_2$ ou CO, soit X représente un groupe CO et Y représente un groupe $CH_2$.

EP 0 524 846 A1

La présente invention a pour objet des dérivés de 2-(pipéridin-1-yl)éthanol, leur préparation et leur application en thérapeutique.

Les composés de l'invention répondent à la formule générale (I)

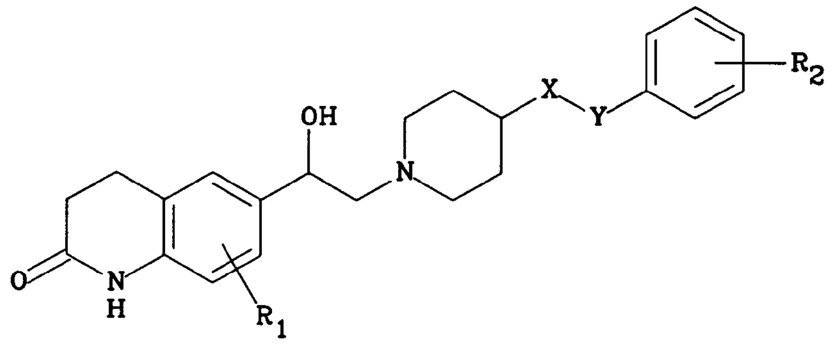

(I)

dans laquelle

$R_1$ représente un atome d'hydrogène ou d'halogène ou un groupe méthyle,

$R_2$ représente un ou deux substituants choisis parmi les atomes d'hydrogène et d'halogènes et les groupes $(C_{1-4})$alkyle, hydroxy, $(C_{1-4})$alcoxy, trifluorométhyle, acétylamino et méthylsulfonylamino, et

soit X représente un groupe $CH_2$ et Y représente un groupe $CH_2$, $(CH_2)_2$ ou CO,

soit X représente un groupe CO et Y représente un groupe $CH_2$, étant exclu le composé dans la formule duquel $R_1$ et $R_2$ représentent chacun un atome d'hydrogène et X et Y représentent chacun un groupe $CH_2$, composé qui est décrit dans la demande de brevet EP-481853.

La molécule représentée par la formule générale (I) possédant un atome de carbone asymétrique, les composés de l'invention peuvent exister sous forme d'énantiomères purs et de mélange d'énantiomères. Enfin, les composés de l'invention peuvent se présenter à l'état de bases libres ou de sels d'addition à des acides pharmaceutiquement acceptables. Ces différentes formes font partie de l'invention.

Les composés de l'invention peuvent être préparés selon le schéma 1 de la page suivante.

## Schéma 1

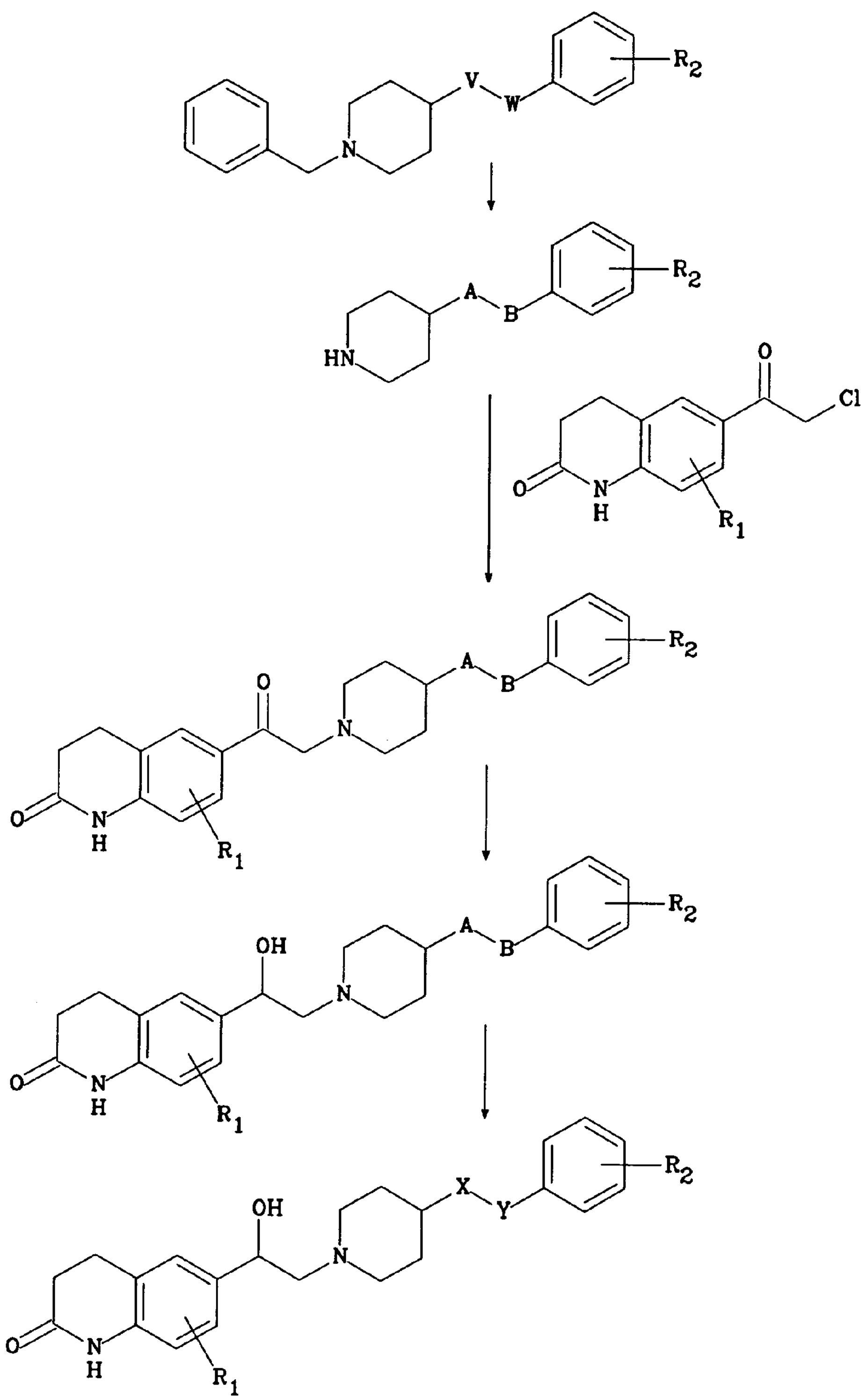

(V)

(II)

(III)

(IV)

(Ia)

(I)

On fait réagir un composé de formule générale (II) dans laquelle

soit A représente un groupe $CH_2$ et B représente un groupe $CH_2$ ou $(CH_2)_2$,
soit A ou B représente un groupe $CH_2$ et l'autre représente un groupe 1,3-dioxolan-2,2-diyle,
avec un composé de formule générale (III), dans laquelle $R_1$ est tel que défini précédemment, pour obtenir un composé de formule générale (IV).

Ensuite, on réduit la fonction cétone du composé (IV) avec un agent réducteur tel que le borohydrure de potassium.
Si A représente un groupe $CH_2$ et B représente un groupe $CH_2$ ou $(CH_2)_2$, le composé de formule (Ia) ainsi obtenu correspond à la formule générale (I) dans laquelle X représente un groupe $CH_2$ et Y représente un groupe $CH_2$ ou $(CH_2)_2$.
Si A ou B représente un groupe $CH_2$ et l'autre représente un groupe 1,3-dioxalan-2,2-diyle, on hydrolyse en milieu acide le composé de formule générale (Ia), et on obtient ainsi un composé de formule générale (I) dans laquelle X ou Y représente un groupe $CH_2$ et l'autre représente un groupe CO.

Les composés de départ de formule générale (II) sont connus ou bien peuvent être préparés selon des méthodes analogues à des méthodes connues ou décrites dans la littérature, par exemple dans *J. Org. Chem.*, **22,** 1376 (1957) ; *C.A.*, **52**, 8138a (1958) ; *C. R. Acad. Sci.*, **255,** 956 (1962) ; brevet US-4254127 ; demandes de brevets EP-12643 et EP-481853; demande de brevet FR-2459795.

Ainsi par exemple on peut partir d'un composé de formule générale (V), dans laquelle V ou W représente un groupe $CH_2$ et l'autre représente un groupe CO.
Pour obtenir un composé de formule générale (II) dans lequel A et B représentent chacun un groupe $CH_2$, on réduit le groupe CO du composé (V) à l'aide d'hydrazine en présence d'une base, puis on déprotège l'azote du cycle de pipéridine par débenzylation.
Pour obtenir un composé de formule générale (II) dans lequel A ou B représente un groupe $CH_2$ et l'autre représente un groupe 1,3-dioxolan-2,2-diyle, on transforme le groupe CO du composé (V) par cétalisation à l'aide d'éthylèneglycol en présence d'acide 4-méthylbenzènesulfonique, puis on déprotège l'azote de la pipéridine par débenzylation.

Les composés de formule générale (II) dans laquelle A et B représentent chacun un groupe $CH_2$ et $R_2$ est différent de l'hydrogène sont nouveaux, et font partie de l'invention à titre d'intermédiaires de synthèse. Ils peuvent être préparés selon des procédés illustrés par les schémas 2 et 3 ci-après.

Le schéma 2 illustre un procédé analogue à celui décrit dans *C. R. Acad. Sci.*, **255,** 956 (1962) ; on fait réagir d'abord un ester de formule générale (VI), dans laquelle Alk représente un groupe $(C_{1-4})$ alkyle avec un benzèneacétonitrile de formule générale (VII) dans laquelle $R_2$ est tel que défini ci-dessus, en présence d'amidure de sodium. On obtient un énol de formule générale (VIII) que l'on traite en milieu acide, pour obtenir une cétone de formule générale (IX), qu'on réduit par exemple au moyen d'hydrate d'hydrazine en présence de potasse et de triéthylèneglycol, pour obtenir un composé de formule générale (X), et finalement on soumet ce dernier à une débenzylation par hydrogénation catalytique ou par déprotection chimique par réaction avec le chloroformiate de trichloroéthyle puis traitement du produit résultant avec du zinc dans l'acide acétique, pour obtenir un composé de formule générale (II'), qui correspond à la formule générale (II) lorsque A et B représentent chacun un groupe $CH_2$.

Le schéma 3 illustre un procédé analogue à celui décrit dans *J. Org. Chem.*, **22,** 1376 (1957) ; on fait réagir d'abord la 4-méthylpyridine de formule (XI) avec un benzaldéhyde de formule générale (XII) dans laquelle $R_2$ est tel que défini ci-dessus, dans l'anhydride acétique. On obtient un composé de formule générale (XIII), et on soumet ce dernier à une hydrogénation catalytique.

Schéma 2

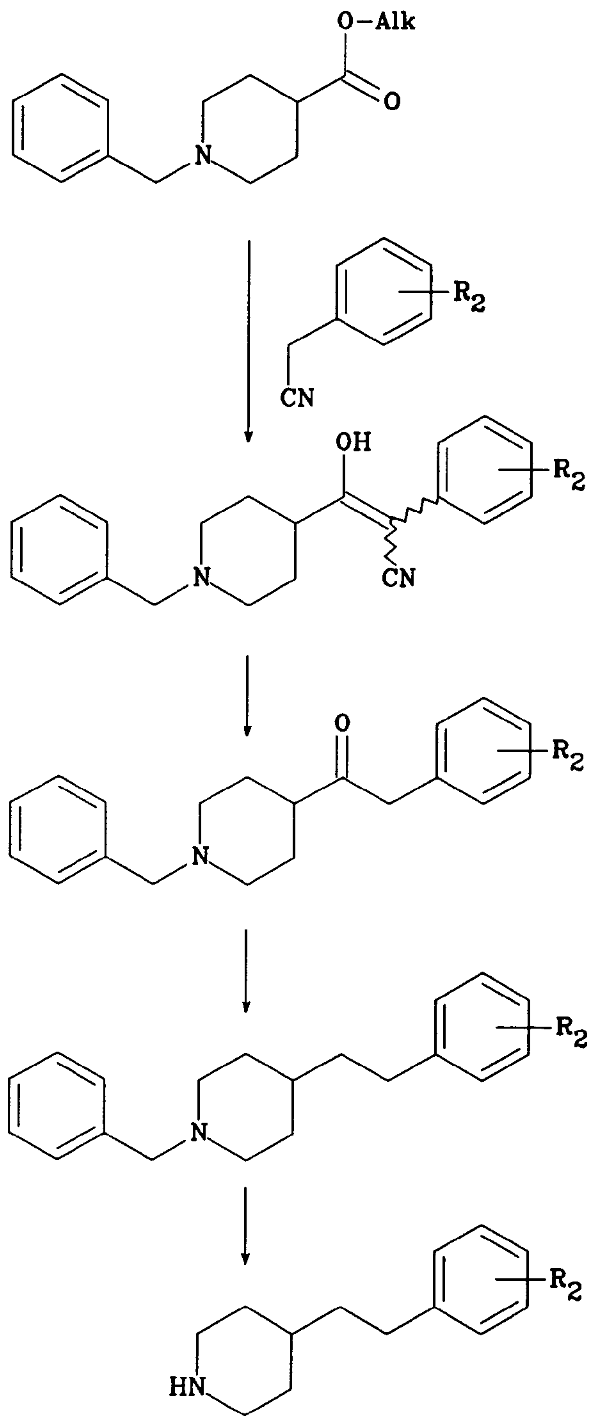

(VI)

(VII)

(VIII)

(IX)

(X)

(II')

Schéma 3

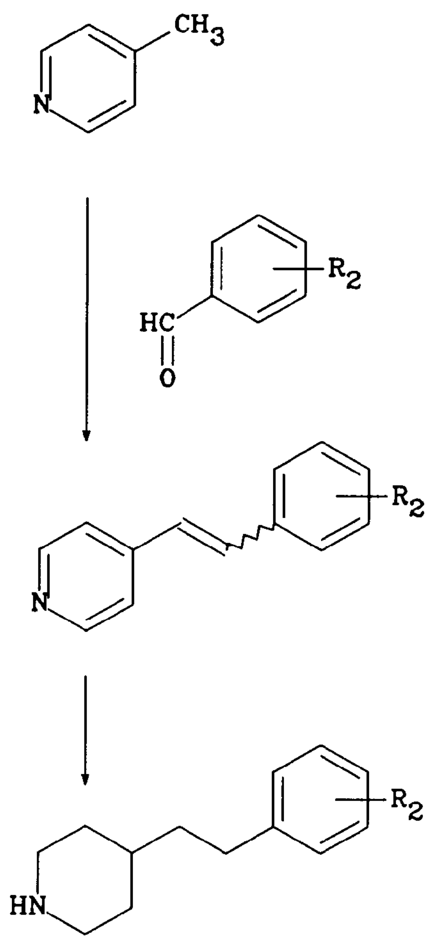

(XI)

(XII)

(XIII)

(II')

Les composés de départ de formules générales (VI), (VII), (XI) et (XII) sont disponibles dans le commerce. Les détails de la préparation de certains composés particuliers sont donnés dans les exemples ci-dessous.

Les composés de départ de formule générale (III), également, sont connus ou peuvent être préparés selon des méthodes analogues à des méthodes connues ou décrites dans la littérature, par exemple dans la demande de brevet EP-0351282 et dans *J. Med. Chem.*, **29,** 2433 (1986).

Ainsi, par exemple, on peut partir d'une quinoléin-2(1*H*)-one convenablement substituée par $R_1$, la soumettre à une hydrogénation catalytique pour obtenir la 3,4-dihydroquinoléin-2(1*H*)-one correspondante, et obtenir le composé de formule générale (III) par action du chlorure d'acide chloroacétique en présence de chlorure d'aluminium.

Enfin, les énantiomères d'un composé selon l'invention peuvent être préparés à partir du racémate selon des méthodes classiques, par exemple par formation d'un sel d'addition avec un acide chiral optiquement pur, cristallisation fractionnée, et libération de la base.

Les exemples suivants illustrent en détail la préparation de quelques composés de l'invention. Les microanalyses et les spectres IR et RMN confirment les structures des composés obtenus.Les numéros des composés indiqués entre parenthèses dans les titres correspondent à ceux des tableaux donnés plus loin.

Les numéros d'exemples et de composés donnés en chiffres romains correspondent à des 4-(2-phényléthyl)pipéridines de départ de formule générale (II'), et les numéros d'exemples et de composés donnés en chiffres arabes correspondent à des composés finals de formule générale (I).

Exemple I (Composé N°III)

4-[2-(4-Fluorophényl)éthyl]pipéridine, chlorhydrate.

I.1. 2-(4-Fluorophényl)-3-hydroxy-3-[1-(phénylméthyl)pipéridin-4-yl]prop-2-ènenitrile.

Dans un ballon de 500 ml, on introduit 13,5 g (0,1 mole) de 4-fluorobenzèneacétonitrile, 50 ml de toluène et 4,4 g d'amidure de sodium. On agite le mélange à température ambiante pendant 30 minutes puis, au moyen d'une ampoule à brome, on ajoute 27,2 g (0,11 mole) de 1-(phénylméthyl)pipéridine-4-carboxylate d'éthyle. L'addition terminée, on chauffe le mélange à 80°C pendant 3 heures. Puis on le refroidit dans un bain de glace, on ajoute 300 ml d'eau, on l'agite 30 minutes, et on ajoute 15 ml d'acide acétique. On obtient un précipité jaune qu'on sépare par filtration, lave à l'eau et sèche en présence de pentoxyde de phosphore. On obtient 17,6 g de composé qu'on utilise tel quel dans l'étape suivante.

I.2. 2-(4-Fluorophényl)-1-[1-(phénylméthyl)pipéridin-4-yl]éthanone.

On place un ballon de 500 ml dans un bain d'eau et de glace, et on introduit 25 ml d'eau, 50 ml d'acide sulfurique concentré, 50 ml d'acide acétique, et on ajoute 24,4 g (0,072 mole) de 2-(4-fluorophényl)-3-hydroxy-3-[1-(phénylméthyl)pipéridin-4-yl]prop-2-ènenitrile. On chauffe au reflux pendant 12 heures. On refroidit le ballon dans un bain d'eau et de glace. On introduit, dans un erlenmeyer de 1 litre, 200 ml de glace, on ajoute lentement le mélange préparé précédemment, on ajoute 200 ml d'acétate d'éthyle et 200 ml d'ammoniaque pour ajuster le pH à 9. On décante, on recueille la phase organique et on extrait la phase aqueuse trois fois avec 150 ml d'acétate d'éthyle. On réunit les phases organiques, on les lave trois fois avec 150 ml d'eau, jusqu'à obtenir un pH de 7 à 8. On sèche la phase organique sur sulfate de sodium, on la filtre et évapore l'acétate d'éthyle sous pression réduite. On obtient 20,7 g de composé huileux qu'on utilise tel quel dans l'étape suivante.

I.3. 4-[2-(4-Fluorophényl)éthyl]-1-(phénylméthyl)pipéridine.

Dans un ballon de 1 litre, on introduit 62 g (0,199 mole) de 2-(4-fluorophényl)-1-[1-(phénylméthyl)pipéridin-4-yl]éthanone en solution dans 400 ml d'éthanol. On ajoute 11,6 ml (0,23 mole) d'hydrate d'hydrazine et on chauffe au reflux pendant 2,5 heures. On refroidit dans un bain de glace, puis on concentre la solution à moitié. Le produit cristallise. On ajoute alors 400 ml d'eau, on agite, et on filtre le précipité blanc. On essore ce précipité, on le sèche au dessicateur en présence de pentoxyde de phosphore. On obtient 73,3 g d'hydrazone intermédiaire. On en reprend 64,8 g (environ 0,199 mole) dans 300 ml de triéthylèneglycol, et on ajoute 30 g d'hydroxyde de potassium. On porte rapidement à 200°C, pendant 1 heure. On refroidit rapidement dans la glace. On ajoute ensuite 600 ml d'eau et on extrait trois fois à l'acétate d'éthyle. On lave la phase organique avec une solution aqueuse saturée de chlorure de sodium. On la sèche sur sulfate de sodium, puis on évapore le solvant sous pression réduite. On obtient une huile marron foncée qu'on purifie par chromatographie sur colonne de gel de silice. On obtient 36,68 g d'huile claire, qu'on utilise telle quelle dans l'étape suivante.

I.4. 4-[2-(4-Fluorophényl)éthyl]pipéridine, chlorhydrate.

On introduit, dans un appareil de Parr, 9,8 g (environ 0,033 mole) de 4-[2-(4-fluorophényl)éthyl]-1-(phénylméthyl)pipéridine en solution dans 100 ml d'éthanol et 10 ml de solution aqueuse normale d'acide chlorhydrique. On ajoute 1 g de palladium sur charbon à 10%. On effectue une hydrogénation à 0,35 MPa et à 50°C pendant 6 heures. On élimine le catalyseur par filtration, on le lave à l'eau et à l'éthanol, et on ajoute 2 ml d'acide chlorhydrique concentré. On évapore le mélange, on le sèche azéotropiquement avec un mélange éthanol/toluène. On évapore à nouveau et on reprend le résidu par 100 ml de tétrahydrofurane. On agite 15 minutes et on observe un début de précipitation. On ajoute 20 ml d'éther diisopropylique et on agite encore 15 minutes. On filtre rapidement le solide puis on le sèche au dessicateur en présence de pentoxyde de phosphore. On obtient 5 g de composé.
Point de fusion : 131-132°C.

<u>Exemple II</u> (Composé N°IV)

4-[2-(4-Chlorophényl)éthyl]pipéridine, acétate.

II.1. 2-(4-Chlorophényl)-3-hydroxy-3-[1-(phénylméthyl)pipéridin-4-yl]prop-2-ènenitrile.

Dans un ballon de 500 ml, on introduit 30,3 g (0,2 mole) de 4-chlorobenzèneacétonitrile, 100 ml de toluène et 8,8 g d'amidure de sodium. On agite le mélange à température ambiante pendant 30 minutes puis, au moyen d'une ampoule à brome, on ajoute 49,4 g (environ 0,2 mole) de 1-(phénylméthyl)pipéridine-4-carboxylate

d'éthyle dilué dans 40 ml de toluène. L'addition terminée, on chauffe le mélange à 80°C pendant 3 heures. Puis on le refroidit dans un bain de glace, on ajoute 800 ml d'eau, on l'agite 30 minutes, et on ajoute 15 ml d'acide acétique. On obtient un précipité qu'on sépare par filtration, lave à l'eau et sèche en présence de pentoxyde de phosphore. On obtient 65,67 g de composé qu'on utilise tel quel dans l'étape suivante.

II.2. 2-(4-Chlorophényl)-1-[1-(phénylméthyl)pipéridin-4-yl]éthanone.

On place un ballon de 500 ml dans un bain d'eau et de glace, et on introduit 50 ml d'eau, 100 ml d'acide sulfurique concentré puis 100 ml d'acide acétique et, tout en agitant, on ajoute 30,21 g (0,086 mole) de 2-(4-chlorophényl)-3-hydroxy-3-[1-(phénylméthyl)pipéridin-4-yl]prop-2-ènenitrile. On chauffe au reflux pendant 12 heures. On introduit, dans un erlenmeyer de 1 litre, 200 ml de glace, on ajoute lentement le mélange préparé précédemment, on ajoute 400 ml d'ammoniaque pour ajuster le pH entre 8 et 9. On agite le mélange dans un bain de glace, on sépare le précipité formé par filtration, on le lave à l'eau et on le sèche en présence de pentoxyde de phosphore. On obtient 28,75 g de composé qu'on utilise tel quel dans l'étape suivante.

II.3. 4-[2-(4-Chlorophényl)éthyl]-1-(phénylméthyl)pipéridine.

Dans un ballon de 500 ml on introduit 26,26 g (0,08 mole) de 2-(4-chlorophényl)-1-[1-(phénylméthyl)pipéridin-4-yl]éthanone en solution dans 160 ml d'éthanol. On ajoute 4,7 ml (0,093 mole) d'hydrate d'hydrazine et on chauffe au reflux pendant 1,5 heure. On refroidit dans un bain de glace, puis on concentre la solution à moitié. Le produit cristallise. On ajoute alors 160 ml d'eau et 200 ml d'acétate d'éthyle, on agite, on sépare la phase organique, on extrait encore une fois la phase aqueuse, on lave la phase organique avec une solution aqueuse saturée de chlorure de sodium, on la sèche sur sulfate de sodium, on évapore le solvant sous pression réduite, et on obtient 28,3 g d'hydrazone intermédiaire sous forme d'huile qui cristallise. On la reprend avec 130 ml de triéthylèneglycol, et on ajoute 14 g d'hydroxyde de potassium. On porte rapidement à 200°C, pendant 1 heure. On refroidit rapidement dans la glace. On ajoute 260 ml d'eau et on extrait cinq fois à l'acétate d'éthyle. On lave la phase organique avec une solution aqueuse saturée de chlorure de sodium. On la sèche sur sulfate de sodium, puis on évapore le solvant sous pression réduite. On obtient 27,07 g d'une huile marron qu'on utilise telle quelle dans l'étape suivante.

II.4. 4-[2-(4-Chlorophényl)éthyl]pipéridine, acétate.

Dans un ballon de 500 ml on introduit 10,7 g (0,034 mole) de 4-[2-(4-chlorophényl)éthyl]-1-(phénylméthyl)pipéridine, 14,4 g (0,068 mole) de chloroformiate de trichloroéthyle, 0,5 g de carbonate de potassium et 100 ml de toluène. On chauffe le mélange au reflux pendant 5 heures, on le laisse refroidir, on ajoute 200 ml d'eau, on agite, on sépare la phase aqueuse, on l'extrait à l'acétate d'éthyle, on lave la phase organique avec une solution aqueuse saturée de chlorure de sodium, on la sèche sur sulfate de sodium et on évapore le solvant sous pression réduite. On obtient 21,7 g d'une huile qu'on introduit dans un ballon de 500 ml, on ajoute 150 ml d'acide acétique et 9,13 g (0,139 mole) de zinc en poudre, et on agite le mélange pendant une nuit. On sépare le zinc par filtration, on évapore le filtrat sous pression réduite, on triture le résidu avec de l'éther diéthylique, on sépare le solide par filtration, on le lave à l'éther diéthylique et on le sèche en présence de pentoxyde de phosphore. On isole finalement 7,6 g de produit brut.
Point de fusion : 155°C.

<u>Exemple III</u> (Composé N°VII)

4-[2-(4-Méthoxyphényl)éthyl]pipéridine, chlorhydrate.

III.1. 4-[2-(4-Méthoxyphényl)éthényl]pipéridine.

On introduit dans un ballon 4656 g, soit 48,6 ml (0,5 mole) de 4-méthylpyridine, 122,5 g, soit 109,5 ml (0,9 mole) de 4-méthoxybenzaldéhyde et 50 ml d'anhydride acétique, et on chauffe le mélange à 180°C pendant 3,5 heures. On le laisse reposer à température ambiante pendant une nuit, on concentre sous pression réduite. On lave le résidu plusieurs fois avec de l'éther diéthylique et on le sèche en présence de pentoxyde de phosphore. On obtient 35 g de composé qu'on utilise tel quel dans l'étape suivante.
Point de fusion : 122-123°C.

III.2. 4-[2-(4-Méthoxyphényl)éthyl]pipéridine, chlorhydrate.

On introduit dans un flacon de Parr 35 g (0,166 mole) de 4-[2-(4-méthoxyphényl)éthényl]pipéridine, un mélange de 175 ml d'éthanol et 175 ml d'acide chlorhydrique 1N et 3 g de charbon palladié à 10%, et on effectue une hydrogénation sous environ 0,35 MPa à 50°C pendant 14 heures. On sépare le catalyseur par filtration, en le lavant à l'éthanol, et on évapore le solvant sous pression réduite. On élimine les traces d'eau du résidu par entraînement azéotropique avec du toluène, on lave le résidu avec de l'éther diéthylique et on le sèche en présence de pentoxyde de phosphore. On obtient 36,08 g de composé. On en recristallise 3 g dans le propanol-2 et on sèche le produit à 80°C en présence de pentoxyde de phosphore. On obtient 2,53 g de composé.
Point de fusion : 178-179°C.

Exemple IV (Composé N°VIII)

4-[2-(4-Trifluorométhylphényl)éthyl]pipéridine, chlorhydrate.

IV.1. 4-[2-(4-Trifluorométhylphényl)éthényl]pipéridine.

On introduit dans un ballon 46,56 g, soit 48,6 ml (0,5 mole) de 4-méthylpyridine, 104,47 g, soit 81,9 ml (0,6 mole) de 4-trifluorométhylbenzaldéhyde et 50 ml d'anhydride acétique, et on chauffe le mélange à 180°C pendant 7,25 heures. On concentre sous pression réduite. On triture le résidu dans 100 ml d'éther isopropylique, on l'essore et on le sèche en présence de pentoxyde de phosphore. On obtient 73,65 g de composé qu'on utilise tel quel dans l'étape suivante.
Point de fusion : 72°C.

IV.2. 4-[2-(4-Trifluorométhylphényl)éthyl]pipéridine, chlorhydrate.

On introduit dans un flacon de Parr 73,15 g (0,293 mole) de 4-[2-(4-trifluorométhylphényl)éthényl]pipéridine, un mélange de 350 ml d'éthanol et 350 ml d'acide chlorhydrique 1N et 5 g de charbon palladié à 10%, et on effectue une hydrogénation sous environ 0,35 MPa à 50°C pendant 8 heures. On sépare le catalyseur par filtration, on le lave à l'éthanol, et on évapore le solvant sous pression réduite, on lave le résidu avec de l'éther diéthylique et on le sèche en présence de pentoxyde de phosphore. On obtient 37,2 g de composé. On en recristallise 3 g dans 30 ml de toluène, on le reprend avec 30 ml d'éther diéthylique, on l'essore et on le sèche à 80°C en présence de pentoxyde de phosphore. On obtient 2,72 g de composé.
Point de fusion : 166-167°C.

Exemple V (Composé N°XIV)

*N*-[4-[2-(pipéridin-4-yl)éthyl]phényl]acétamide, chlorhydrate

V.1. 4-[2-(4-Nitrophényl)éthényl]pyridine.

On introduit dans un ballon 15,11 g (0,1 mole) de 4-nitro-benzaldéhyde, 9,3 g (0,1 mole) de 4-méthylpyridine et 15 ml d'anhydride acétique, et on chauffe le mélange dans un bain d'huile (température du bain : 180°C) pendant 7 heures. On concentre sous pression réduite, on reprend le résidu avec de l'éther de pétrole et on le triture. On sépare le solide par filtration et on le sèche.

V.2. 4-[2-(Pyridin-4-yl)éthyl]benzénamine.

On introduit dans un flacon de Parr le produit de l'étape précédente, 140 ml d'éthanol, 100 ml d'acide chlorhydrique 1N et 3 g de charbon palladié à 10%, et on effectue une hydrogénation sous 0,28 MPa pendant 8 heures à température ambiante. On sépare le catalyseur par filtration, on évapore le filtrat, on sèche le résidu par entraînement au toluène et on le triture dans de l'éther diéthylique.
Après séchage on obtient 10,3 g de produit.
Point de fusion : 108-110°C.

V.3. *N*-[4-[2-(pyridin-4-yl)éthyl]phényl]acétamide.

On introduit dans un ballon 5 g (0,025 mole) de 4-[2-(pyridin-4-yl)éthyl]benzénamine et 25 ml de dichlorométhane, on agite le mélange pendant 5 minutes à température ambiante pour obtenir une solution, on ajoute 10 ml d'anhydride acétique, et on agite le mélange pendant 2 heures à température ambiante.

On sépare le précipité par filtration, on le reprend avec 50 ml d'eau, on ajoute de l'ammoniaque diluée, on agite pendant 30 minutes, on sépare le précipité blanc par filtration, on le lave à l'eau et on le sèche en présence de pentoxyde de phosphore.

On obtient 5,35 g de composé.

Point de fusion : 178°C.

V.4. *N*-[4-[2-(pipéridin-4-yl)éthyl]phényl]acétamide, chlorhydrate .

On introduit dans un flacon de Parr 5,25 g de *N*-[4-[2-(pyridin-4-yl)éthyl]phényl]acétamide en solution dans un mélange de 50 ml d'éthanol et 25 ml d'acide chlorhydrique 1N, on ajoute 0,5 g de charbon palladié à 10%, et on effectue une hydrogénation sous 0,35 MPa à 50°C pendant 16 heures.

On sépare le catalyseur par filtration, on évapore les solvants, on reprend le résidu avec un mélange d'éthanol et d'éther diéthylique et on le triture.

Après filtration et séchage en présence de pentoxyde de phosphore on obtient 5,2 g de produit.

On en recristallise 1 g dans du propanol à 1% d'acide chlorhydrique concentré.

On obtient 0,68 g de composé.

Point de fusion : 256-258°C.

Exemple VI (Composé N°XI)

4-[2-(3,5-Difluorophényl)éthyl]pipéridine, chlorhydrate.

VI.1. 4-[2-(3,5-Difluorophényl)éthényl]pyridine.

On introduit dans un ballon 25 g (0,176 mole) de 3,5-difluorobenzaldéhyde, 16,37 g (0,176 mole) de 4-méthylpyridine et 20 ml d'acide acétique, et on chauffe le mélange dans un bain d'huile (température du bain: 180°C) pendant 7 heures. On évapore le solvant sous pression réduite, et on obtient une huile qui cristallise en refroidissant. On reprend ce résidu avec 50 ml d'éther diisopropylique, on triture le mélange, on sépare le solide par filtration, on le lave avec de l'éther diisopropylique, et on le sèche en présence de pentoxyde de phosphore.

On obtient 30,94 g de produit.

Point de fusion : 114°C.

VI.2. 4-[2-(3,5-Difluorophényl)éthyl]pipéridine, chlorhydrate.

On introduit dans un flacon de Parr 30,90 g (0,142 mole) de 4-[2-(3,5-difluorophényl)éthényl]pyridine, 140 ml d'éthanol, 140 ml d'acide chlorhydrique 1N et 2,5 g de charbon palladié à 10%, et on effectue une hydrogénation sous 0,35 Mpa pendant 14 heures.

On sépare le catalyseur par filtration, on évapore le filtrat, on sèche le résidu par entraînement au toluène puis en présence de pentoxyde de phosphore.

On obtient 30,88 g de composé. On en purifie 1,5 g par recristallisation dans 60 ml de toluène, lavage à l'éther diéthylique, puis séchage en présence de pentoxyde de phosphore. On isole finalement 1,28 g de chlorhydrate pur.

Point de fusion : 161-162°C.

Le tableau 1 ci-après illustre les structures chimiques et les propriétés physiques de quelques composés de formule générale (II').

Dans la colonne "Sel", HCl désigne un chlorhydrate et AcOH désigne un acétate ; dans la colonne "F (°C)", * désigne un produit brut, c'est-à-dire non purifié et dont la microanalyse élémentaire n'a pas encore confirmé la structure.

Tableau 1

4-(2-phényléthyl)pipéridines de formule générale (II')

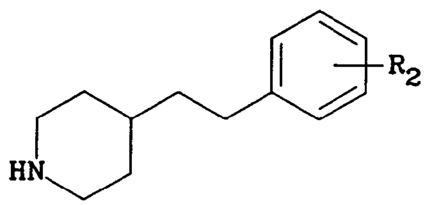

| N° | $R_2$ | Sel | F (°C) |
|---|---|---|---|
| I | 2-F | HCl | 151-152 |
| II | 3-F | HCl | 171-172 |
| III | 4-F | HCl | 131-132 |
| IV | 4-Cl | AcOH | 155* |
| V | 4-$CH_3$ | HCl | 160-161 |
| VI | 3-$CH_3$ | HCl | 119-120 |
| VII | 4-$OCH_3$ | HCl | 178-179 |
| VIII | 4-$CF_3$ | HCl | 166-167 |
| IX | 2-$CF_3$ | HCl | 186-187 |
| X | 3-$CF_3$ | HCl | 73-74 |
| XI | 3,5-$F_2$ | HCl | 161-162 |
| XII | 2,4-$F_2$ | HCl | 130-131 |
| XIII | 3,4-$F_2$ | HCl | 137-138 |
| XIV | 4-$NHCOCH_3$ | HCl | 256-258 |

Exemple 1 (Composé N°3)

(±)-6-[1-Hydroxy-2-[4-[2-(4-fluorophényl)éthyl]pipéridin-1-yl]éthyl]-3,4-dihydroquinoléin-2(1*H*)-one.

On place dans un ballon 4,5 g (0,02 mole) de 6-(chloroacétyl)-3,4-dihydroquinoléin-2(1*H*)-one, 100 ml d'éthanol et 20 ml d'eau. On ajoute 4,9 g (0,02 mole) de chlorhydrate de 4-[2-(4-fluorophényl)éthyl]pipéridine, puis 4 g (environ 0,04 mole) de carbonate de sodium. On chauffe au reflux pendant 1,25 heure. On laisse refroidir, puis on ajoute 9 g de borohydrure de potassium. On agite encore 2 heures puis on laisse le mélange pendant une nuit à température ambiante. On ajoute ensuite 200 ml d'eau, on agite 1 heure et on filtre le précipité formé. On le lave soigneusement à l'eau, puis on l'essore et on le sèche au dessicateur en présence de pentoxyde de phosphore. On purifie le produit obtenu par chromatographie sur gel de silice, en éluant avec un mélange 9/1 de dichlorométhane/méthanol, puis on le recristallise dans 90 ml d'éthanol. On le sèche au dessicateur en présence de pentoxyde de phosphore, puis sous vide à 80°C. On obtient 5,33 g de composé.
Point de fusion : 159-160°C.

Exemple 2 (Composé N°4)

(±)-6-[1-Hydroxy-2-[4-[2-(4-chlorophényl)éthyl]pipéridin-1-yl]éthyl]-3,4-dihydroquinoléin-2(1*H*)-one.

2.1. 4-[2-(4-Chlorophényl)éthyl]pipéridine, chlorhydrate.

Dans un ballon, on introduit 25,14 g (0,08 mole) de 4-[2-(4-chlorophényl)éthyl]-1-(phénylméthyl)pipéridine en solution dans 100 ml de dichlorométhane anhydre, et 8,4 ml (0,088 mole) de chloroformiate d'éthyle. On chauffe au reflux pendant 3,5 heures. On évapore le solvant et on purifie le résidu par chromatographie sur colonne de gel de silice, en éluant avec de l'éther isopropylique. On récupère une huile claire, à laquelle on ajoute 50 ml d'une solution aqueuse de soude à 30% en volume et 100 ml d'éthanol. On chauffe le mélange à 80°C pendant 26 heures. On évapore à sec puis on reprend le résidu avec une solution de 100 ml d'eau et 100 ml d'une solution aqueuse saturée de chlorure de sodium. On extrait trois fois à l'acétate d'éthyle puis on lave la phase organique avec une solution aqueuse saturée de chlorure de sodium jusqu'à obtenir un pH neutre. On ajoute à la phase organique 5 ml d'acide chlorhydrique concentré, puis on l'évapore à sec : un produit cristallise. On effectue un entrainement azéotropique à l'éthanol, on reprend le précipité par de l'éther, on filtre, on lave le précipité à l'éther puis on le sèche au dessicateur en présence de pentoxyde de phosphore. On obtient 8,35 g de chlorhydrate qu'on utilise tel quel dans l'étape suivante.

2.2. (±)-6-[1-Hydroxy-2-[4-[2-(4-chlorophényl)éthyl]pipéridin-1-yl]éthyl]-3,4-dihydroquinoléin-2(1*H*)-one.

On place, dans un ballon de 500 ml, 3,35 g (0,015 mole) de 6-chloroacétyl-3,4-dihydroquinolein-2(1*H*)-one, 3,90 g (0,015 mole) de chlorhydrate de 4[2-(4-chlorophényl)éthyl]pipéridine et 4,24 g (0,04 mole) de carbonate de sodium dans une solution de 80 ml d'éthanol et 20 ml d'eau. On chauffe au reflux pendant 1 heure. On laisse refroidir à température ambiante, puis on ajoute 7,25 g de borohydrure de potassium. On agite, puis on laisse le mélange pendant une nuit. On ajoute ensuite 160 ml d'eau, on agite, et on collecte le précipité par filtration. On le lave à l'eau, on l'essore et on le sèche au dessicateur en présence de pentoxyde de phosphore. On recristallise le produit obtenu dans 350 ml d'éthanol, puis on le recristallise à nouveau dans 80 ml de propanol. On obtient 4,12 g de composé.
Point de fusion : 189-190°C.

Exemple 3 (Composé N°18)

(±)-8-Fluoro-6-[1-hydroxy-2-[4-[2-(4-fluorophényl)éthyl]-pipéridin-1-yl]éthyl]-3,4-dihydroquinoléin-2(1*H*)-one.

On place dans un ballon 3,6 g (0,015 mole) de 6-(chloroacétyl)-8-fluoro-3,4-dihydroquinoléin-2(1*H*)-one, 3,65 g (0,015 mole) de chlorhydrate de 4-[2-(4-fluorophényl)éthyl]pipéridine et 3 g (environ 0,03 mole) de carbonate de sodium dans un mélange de 80 ml d'éthanol et 20 ml d'eau. On chauffe au reflux pendant 1,25 heure. On refroidit puis on ajoute 7 g de borohydrure de potassium. On agite pendant 3 heures puis on laisse le mélange pendant une nuit. On ajoute 160 ml d'eau, on agite 30 minutes, puis on collecte le précipité par filtration. On le lave à l'eau, on le sèche au dessicateur en présence de pentoxyde de phosphore puis on le purifie par chromatographie sur colonne de gel de silice, en éluant avec un mélange 9/1 de dichlorométhane/méthanol. On recristallise le produit dans 70 ml de propan-2-ol puis on le sèche à 80°C en présence d'hydroxyde de potassium. On obtient 2,93 g de composé.
Point de fusion : 163-164°C.

Exemple 4 (Composé N°10)

(±)-6-[1-Hydroxy-2-[4-(3-phénylpropyl)pipéridin-1-yl]éthyl]-3,4-dihydroquinoléin-2(1*H*)-one.

Dans un ballon de 500 ml on place 3,35 g (0,015 mole) de 6-(chloroacétyl)-3,4-dihydroquinoléin-2(1*H*)-one en solution dans 100 ml d'éthanol et 20 ml d'eau, 3,05 g (0,015 mole) de 4-(3-phénylpropyl)pipéridine, et

2,12 g (0,02 mole) de carbonate de sodium. On chauffe au reflux pendant 1 heure. On laisse refroidir à température ambiante, puis on ajoute 6,4 g de borohydrure de potassium. On agite, puis on laisse le mélange pendant 1 nuit à température ambiante. On ajoute 200 ml d'eau et on agite, on filtre le précipité formé, on le lave soigneusement à l'eau, on le sèche au dessicateur en présence de pentoxyde de phosphore, puis on le recristallise dans l'éthanol. Après séchage on obtient 4,04 g de composé.
Point de fusion : 164-165°C.

<u>Exemple 5</u> (Composé N°11)

(±)-6-[1-Hydroxy-2-[4-(2-phényl-2-oxoéthyl)pipéridin-1-yl]-éthyl]-3,4-dihydroquinoléin-2(1*H*)-one.

5.1. 1-Phényl-2-[1-(phénylméthyl)pipéridin-4-yl]éthanone.

Dans un ballon de 500 ml on introduit 2,35 g (0,0966 at.-g) de magnésium en tournures et 40 ml d'éther diéthylique, on agite lentement le mélange et on ajoute 1 ml de bromobenzène puis un cristal d'iode. On augmente l'agitation et, à la température du reflux, on ajoute 9,2 ml de bromobenzène (soit un total de 0,0966 mole) dans 20 ml d'éther diéthylique. On maintient le reflux pendant 30 minutes, puis on laisse refroidir le mélange à température ambiante. On ajoute alors doucement une solution de 10 g (0,0466 mole) de 1-(phénylméthyl )pipéridine-4-acétonitrile dans 10 ml d'éther diéthylique, en chauffant de manière à maintenir un léger reflux. On maintient le reflux pendant 3,25 heures. On refroidit le mélange dans un bain d'eau glacée, on ajoute lentement 100 ml d'eau puis 50 ml d'acide chlorhydrique 5N et on l'agite pendant 1,5 heure à température ambiante. Il se forme un précipité qu'on recueillle par filtration et qu'on lave à l'eau. Après séchage en présence de pentoxyde de phosphore on obtient 15,8 g de produit qu'on utilise tel quel dans l'étape suivante.

5.2. 4-[(2-Phényl-1,3-dioxolan-2-yl)méthyl]-1-(phénylméthyl)pipéridine.

On introduit dans un ballon 15,8 g (0,0466 mole) de 1-phényl-2-[1-(phénylméthyl)pipéridine-4-yl]éthanone en solution dans 150 ml de toluène. On ajoute 30 g (27 ml) d'éthylèneglycol et 16 g d'acide 4-méthylbenzènesulfonique. On chauffe au reflux avec un appareil de Dean-Stark pendant 3,5 heures. On évapore le toluène sous pression réduite, on ajoute 150 ml d'acétate d'éthyle, puis 50 ml d'une solution d'ammoniaque à 30% en volume. On agite, on décante et on extrait un seconde fois à l'acétate d'éthyle, on réunit les phases organiques, on lave avec une solution saturée de chlorure de sodium, on sèche sur sulfate de sodium, et on évapore le solvant sous pression réduite. On obtient 14,56 g d'une huile qu'on utilise telle quelle dans l'étape suivante.

5.3. 4-[(2-Phényl-1,3-dioxolan-2-yl)méthyl]pipéridine.

On introduit dans un appareil de Parr 14,56 g (0,043 mole) de 4-[(2-phényl-1,3-dioxolan-2-yl)méthyl]-1-(phénylméthyl)pipéridine en solution dans 100 ml d'éthanol et 20 ml d'eau. On ajoute 1 g de palladium sur charbon à 10%. On hydrogène à 0,35 MPa et à 50°C pendant 5 heures. On sépare le catalyseur par filtration, on le lave à l'eau et à l'éthanol, on recueille le filtrat et on évapore le solvant sous pression réduite. On obtient 10,26 g d'une huile qu'on utilise telle quelle dans l'étape suivante.

5.4. (±)-6-[1-Hydroxy-2-[4-[(2-phényl-1,3-dioxolan-2-yl)méthyl]pipéridin-1-yl]éthyl]-3,4-dihydroquinoléin-2(1*H*)-one.

Dans un ballon de 500 ml on place 5,13 g (0,0207 mole) de 4-[(2-phényl-1,3-dioxolan-2-yl)méthyl]pipéridine en solution dans 100 ml d'éthanol et 20 ml d'eau, 4,64 g (0,0207 mole) de 6-(chloroacétyl)-3-,4-dihydroquinoléin-2(1*H*)-one et 2,2 g (0,0207 mole) de carbonate de sodium. On chauffe à 80°C pendant 1,25 heure. On laisse refroidir à température ambiante, puis on ajoute 9,8 g de borohydrure de potassium. On continue à agiter à température ambiante, puis on laisse le mélange pendant une nuit. On ajoute ensuite 200 ml d'eau. On agite puis on filtre le précipité beige qui s'est formé. On lave ce précipité à l'eau, et on l'essore. On obtient 7,54 g de composé humide, qu'on utilise tel quel dans l'étape suivante.

5.5. (±)-6-[1-Hydroxy-2-[4-(2-phényl-2-oxoéthyl)pipéridin-1-yl]éthyl]-3,4-dihydroquinoléin-2(1*H*)-one.

Dans un ballon de 500 ml on introduit 7,54 g (0,0173 mole) de 6-[1-hydroxy-2-[4-[(2-phényl-1,3-dioxolan-2-yl)méthyl]pipéridin-1-yl]éthyl]-3,4-dihydroquinoléin-2(1*H*)-one. On le met en suspension dans 150 ml de solution aqueuse normale d'acide chlorhydrique, on ajoute 50 ml d'éthanol, on chauffe à 80°C pendant 1 heure,

puis à 100°C pendant 1 heure. On évapore à sec et on obtient un résidu gommeux qu'on reprend avec un mélange de 25 ml d'éthanol, 25 ml d'acétate d'éthyle et 150 ml d'une solution ammoniaquée (26 à 28% d'ammoniaque en volume). On agite à température ambiante pendant 1 heure, puis on filtre le précipité marron obtenu. On le sèche au dessicateur en présence de pentoxyde de phosphore, puis on le purifie par chromatographie sur colonne de gel de silice en éluant avec un mélange 9/1 de dichlorométhane/méthanol. On recristallise le produit obtenu dans 45 ml de propanol. On obtient 1,45 g de composé.
Point de fusion : 186-187°C.

Exemple 6 (Composé N°13)

(±)-6-[1-Hydroxy-2-[4-[2-(4-fluorophényl)-1-oxoéthyl]pipéridin-1-yl]éthyl]-3,4-dihydroquinoléin-2(1*H*)-one.

6.1. 2-(4-Fluorophényl)-3-hydroxy-3-[1-(phénylméthyl)-pipéridin-4-yl]prop-2-ènenitrile.

Dans un ballon de 500 ml, on introduit 13,5 g (0,1 mole) de (4-fluorophényl)acétonitrile, 50 ml de toluène et 4,4 g d'amidure de sodium. On agite le mélange à température ambiante pendant 30 minutes puis, au moyen d'une ampoule à brome, on ajoute 27,2 g (0,11 mole) de 1-(phénylméthyl)pipéridine-4-carboxylate d'éthyle. L'addition terminée, on chauffe le mélange à 80°C pendant 3 heures. Puis on le refroidit dans un bain de glace, on ajoute 300 ml d'eau, on l'agite 30 minutes, et on ajoute 15 ml d'acide acétique. On obtient un précipité jaune qu'on sépare par filtration, lave à l'eau et sèche en présence de pentoxyde de phosphore. On obtient 17,6 g de composé qu'on utilise tel quel dans l'étape suivante.

6.2. 2-(4-Fluorophényl)-1-[1-(phénylméthyl)pipéridin-4-yl]éthanone.

On place un ballon de 500 ml dans un bain d'eau et de glace, et on introduit 25 ml d'eau, 50 ml d'acide sulfurique concentré, 50 ml d'acide acétique, et on ajoute 24,4 g (0,072 mole) de 2-(4-fluorophényl)-3-hydroxy-3-[1-(phénylméthyl)pipéridin-4-yl]prop-2-ènenitrile. On chauffe au reflux pendant 12 heures. On refroidit le ballon dans un bain d'eau et de glace. On introduit, dans un erlenmeyer de 1 litre, 200 ml de glace, on ajoute lentement le mélange précédemment préparé, on ajoute 200 ml d'acétate d'éthyle et 200 ml d'ammoniaque pour ajuster le pH à 9. On décante, on recueille la phase organique et on extrait la phase aqueuse trois fois avec 150 ml d'acétate d'éthyle. On réunit les phases organiques, on les lave trois fois avec 150 ml d'eau, jusqu'à obtenir un pH de 7 à 8. On sèche la phase organique sur sulfate de sodium, on la filtre et évapore l'acétate d'éthyle sous pression réduite. On obtient 20,7 g de composé huileux qu'on utilise tel quel dans l'étape suivante.

6-3. 4-[2-[(4-Fluorophényl)méthyl]-1,3-dioxolan-2-yl]-1-(phénylméthyl)pipéridine.

Dans un ballon de 500 ml muni d'un appareil de Dean-Stark on introduit 10,35 g (0,033 mole) de 2-(4-fluorophényl)-1-[1-(phénylméthyl)pipéridin-4-yl]éthanone, 100 ml de toluène, 20 g d'éthylèneglycol et 10,35 g d'acide 4-méthylbenzènesulfonique, et on chauffe le mélange au reflux pendant 1,75 heure. On évapore le toluène et on reprend le résidu par 200 ml d'acétate d'éthyle. On ajoute 100 ml d'une solution d'ammoniaque à 30%, on décante la phase organique, on la lave trois fois avec 100 ml d'eau, on la sèche, et on évapore le solvant sous pression réduite. On obtient 13,4 g de produit huileux qu'on utilise tel quel dans l'étape suivante.

6.4. 4-[2-[(4-Fluorophényl)méthyl]-1,3-dioxolan-2-yl]pipéridine.

Dans un appareil de Parr on introduit 13,4 g (0,033 mole) de 4-[2-[(4-fluorophényl)méthyl]-1,3-dioxolan-2-yl]-1-(phénylméthyl)pipéridine en solution dans 100 ml d'éthanol et 20 ml d'eau, on ajoute 2 g de palladium sur charbon à 10% et on effectue une hydrogénation à 50°C sous une pression d'hydrogène de 0,35 MPa pendant 5 heures. On sépare le catalyseur par filtration, on le lave à l'eau et à l'éthanol, on évapore le filtrat sous pression réduite et on sèche le résidu sous vide. On obtient 9,74 g de composé huileux qui cristallise et qu'on utilise tel quel dans l'étape suivante.

6.5. (±)-6-[1-Hydroxy-2-[4-[2-(4-fluorophényl)-1,3-dioxolan-2-yl]pipéridin-1-yl]éthyl]-3,4-dihydroquinoléin-2(1*H*)-one.

Dans un ballon de 500 ml on introduit 4,37 g (0,0165 mole) de 4-[2-[(4-fluorophényl)méthyl]-1,3-dioxolan-2-yl]pipéridine en solution dans 80 ml d'éthanol et 20 ml d'eau. On ajoute 3,69 g (0,0165 mole) de 6-(chloroacétyl)-3,4-dihydroquinoléin -2(1*H*)-one et 2 g (environ 0,02 mole) de carbonate de sodium. On chauffe au reflux

pendant 50 minutes. On refroidit le mélange, puis on ajoute 8,56 g de borohydrure de potassium. On continue à agiter à température ambiante, puis on laisse le mélange pendant une nuit à température ambiante. On ajoute 160 ml d'eau et on agite pendant 30 minutes. On sépare le précipité par filtration et on le lave à l'eau. On obtient 8,14 g de produit humide qu'on utilise tel quel dans l'étape suivante.

6.6. (±)-6-[1-Hydroxy-2-[4-[2-(4-fluorophényl)-1-oxoéthyl]pipéridin-1-yl]éthyl]-3,4-dihydroquinoléin-2(1*H*)-one.

Dans un ballon de 500 ml on introduit 7,5 g (0,0165 mole) de 6-[1-hydroxy-2-[4-[2-(4-fluorophényl)-1,3-dioxolan-2-yl]pipéridin-1-yl]éthyl]-3,4-dihydroquinoléin-2(1*H*)-one en suspension dans 150 ml de solution aqueuse normale d'acide chlorhydrique. On ajoute 50 ml d'éthanol, et on agite à 80°C pendant 1 heure. On laisse refroidir, puis on évapore à sec. On reprend le résidu cristallisé avec un mélange de 25 ml d'éthanol, 25 ml d'acétate d'éthyle et 150 ml de solution aqueuse ammoniaquée (26 à 28% d'ammoniaque en volume). On agite à température ambiante pendant 2 heures, on filtre et on reprend le résidu par de l'éthanol. On agite, on ajoute un peu d'eau et on sépare l'insoluble par filtration. On sèche le produit au dessicateur en présence de pentoxyde de phosphore, puis on le purifie par chromatographie sur colonne de gel de silice en éluant avec un mélange 70/15/15 de dichlorométhane/méthanol/acétate d'éthyle. On recristallise le produit dans 10 ml de propanol et on le sèche. On obtient 0,8 g de composé.
Point de fusion : 171-172°C.

<u>Exemple 7</u> (Composé N°15)

(±)-8-Fluoro-6-[1-hydroxy-2-[4-(2-phényléthyl)pipéridinl-1-yl]éthyl]-3,4-dihydroquinoléin-2(1*H*)-one.

7.1. *N*-(2-fluorophényl)-3-phénylprop-2-énamide.

On introduit dans un bain de glace un ballon contenant une solution de 33,3 g (0,3 mole) de 2-fluorobenzèneamine dans 300 ml de toluène. On ajoute 25,5 ml de pyridine, puis, goutte à goutte, une solution de 50 g (0,3 mole) de chlorure de phénylprop-2-énoyle dans 300 ml de toluène. On agite pendant 1 heure en conservant le ballon dans le bain de glace, puis on laisse le mélange à température ambiante pendant une nuit. On filtre le précipité qui s'est formé, on le lave à l'eau puis à l'éther de pétrole, et on le sèche au dessicateur en présence de pentoxyde de phosphore. On obtient 36,2 g de composé.
Point de fusion : 116°C.

7.2. 8-Fluoroquinoléin-2(1*H*)-one.

On met en suspension 34 g (0,141 mole) de *N*-(2-fluorophényl)-3-phénylprop-2-énamide dans 180 ml de chlorobenzène. On ajoute sous agitation 93 g (0,7 mole) de chlorure d'aluminium, par petites fractions. On chauffe ensuite le mélange au reflux pendant 3,5 heures. On le refroidit légèrement et on le verse lentement sur 1 litre de mélange eau/glace, en agitant. On lave le précipité obtenu à l'eau puis à l'éther de pétrole. On le sèche au dessicateur en présence de pentoxyde de phosphore. On obtient 22,5 g de composé.
Point de fusion : 193°C.

7.3. 8-Fluoro-3,4-dihydroquinoléin-2(1*H*)-one.

On introduit 22 g (0,134 mole) de 8-fluoroquinoléin-2(1*H*)-one dans un mélange de 100 ml d'éthanol et 50 ml d'une solution aqueuse normale d'acide chlorhydrique contenant 2 g de palladium sur charbon à 10%, et on effectue une hydrogénation à 50°C, sous une pression de 0,35 MPa, pendant 10 heures. On filtre à chaud la suspension, on lave le catalyseur avec de l'éthanol chaud. Une masse blanche cristallise dans le filtrat. On l'évapore à sec, et on triture le résidu avec un peu d'éther de pétrole. On filtre, on essore, puis on sèche au dessicateur. On obtient 17,85 g de composé.
Point de fusion : 140°C.

7.4. 6-(Chloroacétyl)-8-fluoro-3,4-dihydroquinoléin-2(1*H*)-one.

On place, dans un ballon de 500 ml, 42,4 g (0,318 mole) de chlorure d'aluminium, 30 ml de dichlorométhane, 23,9 g, soit 17 ml (0,212 mole) de chlorure de chloroacétyle. On agite à température ambiante pendant 30 minutes, puis on ajoute, rapidement et par petites fractions, 17,5 g (0,106 mole) de 8-fluoro-3,4-dihydroquinoléin-2(1*H*)-one. On chauffe à 80°C pendant 3,5 heures. On obtient une solution noirâtre qu'on verse lentement sur 1 litre d'un mélange eau/glace. On agite 15 minutes, on collecte le précipité par filtration, on le lave soigneusement à l'eau, on l'essore et on le sèche au dessicateur. On obtient 25 g de composé.
Point de fusion : 194°C.

7.5. (±)-8-Fluoro-6-[1-hydroxy-2-[4-(2-phényléthyl)pipéridin-1-yl]éthyl]-3,4-dihydroquinoléin-2(1*H*)-one.

On place dans un ballon 3,62 g (0,015 mole) de 6-(chloroacétyl)-8-fluoro-3,4-dihydroquinoléin-2(1*H*)-one, 3,38 g (0,015 mole) de chlorhydrate de 4-(2-phényléthyl)pipéridine, 4 g (environ 0,04 mole) de carbonate de sodium. On ajoute 80 ml d'éthanol et 20 ml d'eau. On chauffe au reflux pendant une heure. On refroidit, puis on ajoute 7 g de borohydrure de potassium en une seule fois. On agite à température ambiante pendant 2,5 heures. On ajoute 150 ml d'eau, on agite puis on filtre le précipité formé. On le sèche, on le recristallise dans 50 ml de propan-2-ol, et on le sèche. On obtient 2,5 g de solide.
Point de fusion : 138-140°C.

Exemple 8 (Composé N°17)

(±)-8-Fluoro-6-[1-hydroxy-2-[4-[2-(3-fluorophényl)éthyl]-pipéridin-1-yl]éthyl]-3,4-dihydroquinoléin-2(1*H*)-one.

On place dans un ballon 3,6 g (0,015 mole) de 6-(chloroacétyl)-8-fluoro-3,4-dihydroquinoléin-2(1*H*)-one, 3,6 g (0,015 mole) de chlorhydrate de 4-[2-(3-fluorophényl)éthyl]pipéridine, 3 g (environ 0,03 mole) de carbonate de sodium, et un mélange de 80 ml d'éthanol et 20 ml d'eau. On chauffe au reflux pendant 1,5 heure. On refroidit, puis on ajoute 7 g de boroydrure de potassium. On agite une nuit à température ambiante, on verse le mélange dans 160 ml d'eau et on agite pendant 1 heure. On filtre alors le précipité qui s'est formé, on le lave à l'eau, on l'essore et on le sèche au dessicateur en présence de pentoxyde de phosphore. On obtient 4,9 g de produit qu'on purifie par chromatographie sur colonne de gel de silice en éluant avec une mélange 9/1 de dichlorométhane/méthanol. On obtient 4 g de produit huileux qui cristallise et qu'on recristallise dans 30 ml de propan-2-ol. Après séchage on obtient 2,4 g de composé pur.
Point de fusion : 116-117°C.

Exemple 9 (Composé N°9, 9a et 9b)

(±) 6-[1-Hydroxy-2-[4-[2-[4-(trifluorométhyl)phényl]éthyl]-pipéridin-1-yl]éthyl]-3,4-dihydroquinoléin-2(1*H*)-one et ses énantiomères (-) et (+).

9.1. (±) 6-[1-Hydroxy-2-[4-[2-[4-(trifluorométhyl)phényl]éthyl]pipéridin-1-yl]éthyl]-3,4-dihydroquinoléin-2(1*H*)-one.

Dans un ballon de 1 litre on introduit 22,3 g (0,1 mole) de 6-(chloroacétyl)-3,4-dihydroquinoléin-2(1*H*)-one, 29,3 g (0,1 mole) de chlorhydrate de 4-[2-[4-(trifluorométhyl)phényl]-éthyl]pipéridine, 21,2 g (0,2 mole) de carbonate de sodium, 400 ml d'éthanol et 100 ml d'eau, et on chauffe le mélange au bain d'huile (température du bain : 120°C) pendant 1 heure. On le refroidit avec un bain d'eau froide, on le verse dans 800 ml d'eau, on sépare le précipité marron par filtration, on le lave à l'eau, on prélève 4 g de cette cétone intermédiaire pour d'autres usages.
On reprend le reste avec 400 ml d'éthanol et 100 ml d'eau, on ajoute 80 g de borohydrure de potassium et on agite le mélange pendant deux jours à température ambiante.
On verse le mélange dans 800 ml d'eau, on agite pendant 30 minutes, on filtre le précipité, on le lave à l'eau et on le sèche en présence de pentoxyde de phosphore.
On obtient 34,64 g de produit qu'on recristallise dans 440 ml de propan-2-ol et, après séchage en présence de pentoxyde de phosphore, on obtient 31,18 g de produit.
On en prélève 20 g pour la séparation des énantiomères, et on recristallise les 11,18 g restants une dernière fois dans 120 ml de propan-2-ol.
Après séchage en présence de pentoxyde de phosphore on isole finalement 9,94 g de composé purifié.
Point de fusion : 163-164°C.

9.2. (-) 6-[1-Hydroxy-2-[4-[2-[4-(trifluorométhyl)phényl]éthyl]pipéridin-1-yl]éthyl]-3,4-dihydroquinoléin-2(1*H*)-one.

Dans un erlenmeyer on introduit 20 g (0,0448 mole) de (±) 6-[1-hydroxy-2-[4-[2-[4-(trifluorométhyl)phényl]éthyl]pipéridin-1-yl]éthyl]-3,4-dihydroquinoléin-2(1*H*)-one, 6,8 g (0,0448 mole) d'acide L(+)-mandélique et 100 ml d'éthanol.
On agite le mélange, qui se prend en masse, on le chauffe au reflux et on ajoute de l'éthanol jusqu'à dissolution du sel, soit environ 1500 ml d'éthanol.
On filtre à chaud, on réchauffe le filtrat pour redissoudre le précipité, on ajoute 60 ml d'éthanol pour obtenir une solution limpide, et on l'abandonne, sans agitation, à température ambiante pendant 4 heures.
On filtre les cristaux formés, on les lave avec un peu d'éthanol et on les sèche en présence de pentoxyde de phosphore.
On obtient 14,56 g de produit qu'on recristallise deux fois dans l'éthanol, ce qui laisse 7,09 g de sel.
Point de fusion : 234-234,5°C.
On libère la base de manière classique, dans du dichlorométhane et avec de l'ammoniaque aqueuse. Après séparation de la phase organique et évaporation du solvant on recristallise la base dans 50 ml de propanol et on la sèche en présence de pentoxyde de phosphore.
On isole finalement 4,34 g de composé.
Point de fusion : 172-173°C.
$[\alpha]_D^{20}$ = -34,0° (c = 1,0 ; $CHCl_3$)

9.3. (+) 6-[1-Hydroxy-2-[4-[2-[4-(trifluorométhyl)phényl]éthyl]pipéridin-1-yl]éthyl]-3,4-dihydroquinoléin-2(1*H*)-one.

On rassemble toutes les eaux-mères de l'étape précédente, on évapore le solvant, on ajoute du dichlorométhane et de l'ammoniaque au résidu d'évaporation, on sépare la phase organique et on évapore le solvant.
On obtient 15 g (0,0336 mole) de base qu'on met en suspension dans 100 ml d'éthanol, on ajoute 5,11 g d'acide D(-)-mandélique, on chauffe le mélange au reflux et on ajoute de l'éthanol jusqu'à dissolution du sel, soit environ 1300 ml d'éthanol.
On filtre à chaud, on réchauffe le filtrat pour redissoudre le précipité, et on abandonne la solution, sans agitation, à température ambiante pendant 4 heures.
On filtre les cristaux formés, on les lave avec un peu d'éthanol et on les sèche en présence de pentoxyde de phosphore.
On obtient 10,96 g de produit qu'on recristallise deux fois dans l'éthanol, ce qui laisse 6,11 g de sel.
Point de fusion : 234-235°C.
On libère la base de manière classique, dans du dichlorométhane et avec de l'ammoniaque aqueuse. Après séparation de la phase organique et évaporation du solvant on recristallise la base dans 50 ml de propanol et on la sèche en présence de pentoxyde de phosphore.
On isole finalement 3,78 g de composé.
Point de fusion : 174-175°C.
$[\alpha]_D^{20}$ = +35,0° (c = 1,0 ; $CHCl_3$).

Exemple 10 (Composé N°51)

(±) 6-[1-Hydroxy-2-[4-[2-[4-(acétylamino)phényl]éthyl]pipéridin-1-yl]éthyl]-3,4-dihydroquinoléin-2(1*H*)-one.
On introduit dans un ballon 3,35 g (0,015 mole) de 6-(chloroacétyl)-3,4-dihydroquinoléin-2(1*H*)-one, 4,23 g (0,015 mole) de chlorhydrate de *N*-[4-[2-(pipéridin-4-yl)éthyl]phényl]acétamide, 3,18 g (0,03 mole) de carbonate de sodium, 80 ml d'éthanol et 20 ml d'eau, et on chauffe le mélange au reflux pendant 2 heures.
On laisse refroidir, on ajoute 7 g de borohydrure de potassium, et on agite à température ambiante pendant une nuit.
On ajoute 160 ml d'eau, on agite pendant 30 minutes, on sépare le précipité par filtration, on le lave à l'eau et on le sèche.
On obtient 5,5 g de produit, on le recristallise dans le propanol et on le sèche à 90°C.
On obtient finalement 3,8 g de composé.
Point de fusion : 205-206°C.

Exemple 11 (Composé N°39)

(±) 6-[1-Hydroxy-2-[4-(2-phényléthyl)pipéridin-1-yl]éthyl]-7-fluoro-3,4-dihydroquinoléin-2(1*H*)-one.

11.1. *N*-(3-fluorophényl)-3-phénylprop-2-énamide.

On introduit dans un erlenmeyer, refroidi par un bain de glace, 30 g (0,27 mole) de 3-fluorobenzénamine, 300 ml de toluène et 25 ml de pyridine, puis on ajoute, goutte à goutte, une solution de 45 g (0,27 mole) de chlorure de 3-phénylprop-2-énoyle dans 300 ml de toluène, sans laisser la température dépasser 10°C.
On agite le mélange pendant encore 15 minutes dans le bain de glace, puis pendant une nuit à température ambiante.
On élimine un précipité par filtration, on ajoute 50 ml d'eau au filtrat, et on l'agite vigoureusement pendant 1 heure.
On sépare le précipité par filtration, on le lave à l'eau et on le sèche en présence de pentoxyde de phosphore.
On obtient 51 g de produit.
Point de fusion : 115°C.

11.2. 7-Fluoroquinoléin-2(1*H*)-one.

On introduit dans un ballon 51 g (0,211 mole) de *N*-(3-fluorophényl)-3-phénylprop-2-énamide, 200 ml de chlorobenzène, puis, par petites fractions, 140 g (1,05 mole) de chlorure d'aluminium, on chauffe le mélange au reflux pendant 4 heures et on l'abandonne pendant une nuit.
On le verse sur un mélange d'eau et de glace, on filtre, on lave le solide à l'eau puis à l'éther de pétrole, et on le laisse sécher à l'air libre.
On obtient 34 g de composé.

11.3. 7-Fluoro-3,4-dihydroquinoléin-2(1*H*)-one.

On introduit dans un flacon de Parr 34 g (0,208 mole) de 7-fluoroquinoléin-2(1*H*)-one, 150 ml d'éthanol, 50 ml d'acide chlorhydrique 1N et 3 g de charbon palladié à 10%, et on effectue une hydrogénation sous 0,42 MPa à 50°C pendant 16 heures.
On sépare le catalyseur par filtration, on évapore le filtrat, on triture le résidu blanc avec 50 ml d'éthanol, on le sépare par filtration et on le sèche en présence de pentoxyde de phosphore. On obtient 27,03 g de produit qu'on utilise tel quel dans l'étape suivante.

11.4. 6-(Chloroacétyl)-7-fluoro-3,4-dihydroquinoléin-2(1*H*)-one.

On introduit dans un ballon de 500 ml 65 g (0,49 mole) de chlorure d'aluminium, 50 ml de dichlorométhane, 26 ml (0,327 mole) de chlorure de chloroacétyle, et on agite le mélange à température ambiante pendant 30 minutes.
On ajoute alors, par petites fractions, 27 g (0,163 mole) de 7-fluoro-3,4-dihydroquinoléin-2(1*H*)-one, on chauffe le mélange au reflux pendant 4 heures et on l'abandonne pendant une nuit à température ambiante.
On le verse lentement sur 300 ml d'eau glacée, on agite le mélange pendant 10 minutes, on sépare le précipité par filtration, on le lave à l'eau puis à l'hexane, on l'essore et on le sèche en présence de pentoxyde de phosphore.
On obtient 38 g de produit qu'on utilise tel quel dans l'étape suivante.

11.5. (±) 6-[1-Hydroxy-2-[4-(2-phényléthyl)pipéridin-1-yl]éthyl]-7-fluoro-3,4-dihydroquinoléin-2(1*H*)-one.

On introduit dans un ballon 3,6 g (0,015 mole) de 6-(chloroacétyl)-7-fluoro-3,4-dihydroquinoléin-2(1*H*)-one, 3,38 g de chlorhydrate de 4-(2-phényléthyl)pipéridine, 3,18 g (0,03 mole) de carbonate de sodium, 160 ml d'eau, 40 ml d'eau et 50 ml de *N,N*-diméthylformamide, et on agite le mélange à température ambiante pendant 3 jours.
On ajoute 7 g de borohydrure de potassium et on agite le mélange pendant une nuit à température ambiante.
On le verse dans 350 ml d'eau, on agite pendant 30 minutes, on sépare le précipité par filtration, on le sèche, on le purifie par chromatographie sur colonne de gel de silice en éluant avec un mélange 9/1 de dichlorométhane/méthanol, on le recristallise dans 60 ml de propanol et on le sèche en présence de pentoxyde de phosphore.
On obtient finalement 1,48 g de composé.
Point de fusion : 199-200°C.

Exemple 12 (Composé N°36)

(±) 6-[1-Hydroxy-2-[4-[2-(2-méthylphényl)éthyl]pipéridin-1-yl]éthyl]-8-méthyl-3,4-dihydroquinoléin-2(1*H*)-one.

12.1. *N*-(2-méthylphényl)-3-phénylprop-2-énamide.

On introduit dans un ballon de 2 litres, refroidi par un bain de glace, 32,15 g (0,3 mole) de 2-méthylbenzénamine, 300 ml de toluène et 25,5 ml de pyridine, puis on ajoute, goutte à goutte, une solution de 50 g (0,3 mole) de chlorure de 3-phénylprop-2-énoyle dans 300 ml de toluène.
On retire le bain de glace, puis on agite le mélange pendant 3,5 heures à température ambiante et on l'abandonne pendant une nuit.
On obtient un précipité blanc qu'on sépare par filtration, on le lave plusieurs fois à l'eau puis à l'éther diéthylique, et on le sèche en présence de pentoxyde de phosphore.
On obtient 69,47 g de produit.
Point de fusion : 166°C.

12.2. 8-Méthylquinoléin-2(1*H*)-one.

On introduit dans un ballon de 2 litres 67,5 g (0,284 mole) de *N*-(2-méthylphényl)-3-phénylprop-2-énamide, 370 ml de chlorobenzène, on agite le mélange et, par petites fractions, on ajoute 188,1 g (1,409 mole) de chlorure d'aluminium. On chauffe le mélange au reflux pendant 4 heures et on l'abandonne pendant une nuit.
On le verse lentement sur 1 litre d'eau et de glace, on sépare le précipité marron par filtration, on le lave plusieurs fois à l'eau puis à l'hexane, et on le sèche en présence de pentoxyde de phosphore.
On obtient 42,76 g de composé.
Point de fusion : 224°C.

12.3. 8-Méthyl-3,4-dihydroquinoléin-2(1*H*)-one.

On introduit dans un flacon de Parr 42,76 g (0,266 mole) de 8-méthylquinoléin-2(1*H*)-one, 200 ml d'éthanol, 100 ml d'acide chlorhydrique 1N et 4,2 g de charbon palladié à 10%, et on effectue une hydrogénation sous 0,25 MPa à 50°C pendant 15 heures.
On sépare le catalyseur par filtration à chaud, en le lavant avec de l'éthanol chaud, on évapore le filtrat, on lave le résidu avec de l'éther de pétrole et on le sèche en présence de pentoxyde de phosphore. On obtient 38,15 g de produit qu'on utilise tel quel dans l'étape suivante.
Point de fusion : 131-132°C.

12.4. 6-(Chloroacétyl)-8-méthyl-3,4-dihydroquinoléin-2(1*H*)-one.

On introduit dans un ballon de 500 ml 57,21 g (0,429 mole) de chlorure d'aluminium, 45 ml de dichlorométhane, 32,32 g, soit 22,8 ml, (0,286 mole) de chlorure de chloroacétyle, et on agite le mélange à température ambiante pendant 30 minutes. On ajoute alors, par petites fractions, 23,02 g (0,286 mole) de 8-méthyl-3,4-dihydroquinoléin-2(1*H*)-one, et on chauffe le mélange au reflux pendant 3,5 heures.
On le verse lentement sur 1 litre d'eau glacée, on agite le mélange pendant 10 minutes, on sépare le précipité marron par filtration, on le lave plusieurs fois à l'eau puis à l'éther de pétrole, on l'essore et on le sèche en présence de pentoxyde de phosphore.
On obtient 33,45 g de produit qu'on utilise tel quel dans l'étape suivante.

12.5. (±) 6-[1-Hydroxy-2-[4-[2-(2-méthylphényl)éthyl]pipéridin-1-yl]éthyl]-8-méthyl-3,4-dihydroquinoléin-2(1*H*)-one.

On introduit dans un ballon de 500 ml 3,56 g (0,015 mole) de 6-(chloroacétyl)-8-méthyl-3,4-dihydroquinoléin-2(1*H*)-one, 3,6 g (0,015 mole) de chlorhydrate de 4-[2-(2-méthylphényl)éthyl]pipéridine, 4,24 g (0,04 mole) de carbonate de sodium, 100 ml d'éthanol et 20 ml d'eau, et on chauffe le mélange au reflux pendant 1 heure.
On refroidit le mélange, on ajoute 7,16 g de borohydrure de potassium et on agite le mélange pendant une nuit à température ambiante.
On ajoute 200 ml d'eau, on agite pendant 30 minutes, on sépare le précipité par filtration, on le sèche, on le purifie par chromatographie sur colonne de gel de silice en éluant avec un mélange 9/1 de dichlorométhane/méthanol. On obtient 4,09 g de produit qu'on recristallise dans 130 ml de propanol et on le sèche en présence de

pentoxyde de phosphore. On obtient 3,68 g de produit qu'on recristallise une seconde fois dans 325 ml d'éthanol.
On obtient finalement 3,29 g de composé.
Point de fusion : 185-186°C.

Exemple 13 (Composé N°40)

(±)-6-[1-Hydroxy-2-[4-[2-(3,5-difluorophényl)éthyl]pipéridin-1-yl]éthyl]-3,4-dihydroquinoléin-2(1*H*)-one.

On introduit dans un ballon 3,35 g (0,015 mole) de 6-(chloroacétyl)-3,4-dihydroquinoléin-2(1*H*)-one, 3,9 g (0,015 mole) de chlorhydrate de 4-[2-(3,5-difluorophényl)éthyl]pipéridine, 80 ml d'éthanol et 20 ml d'eau, et on chauffe le mélange au reflux pendant 2 heures.
On le laisse refroidir, on ajoute 7 g de borohydrure de potassium, et on agite une nuit à température ambiante.
On ajoute 160 ml d'eau, on agite pendant 15 minutes, on sépare le solide par filtration, on le lave à l'eau puis à l'hexane, on le sèche et on le purifie par chromatographie sur colonne de gel de silice en éluant avec un mélange 9/1 de dichlorométhane/méthanol.
On obtient 3,7 g de produit qu'on recristallise dans 60 ml d'éthanol. Après séchage on isole finalement 3,08 g de composé.
Point de fusion : 173-174°C.

Le tableau suivant illustre les structures chimiques et propriétés physiques de quelques composés de formule générale (I).
Tous les composés sont à l'état racémique, à l'exception des composés N°9a et N°9b, qui sont les énantiomères respectivement lévogyre et dextrogyre du composé N°9.
Dans la colonne "Sel", "-" désigne un composé à l'état de base, "fum." désigne un composé à l'état de fumarate, et "ox." désigne un composé à l'état d'oxalate.

Tableau 2
Composés de formule générale (I)

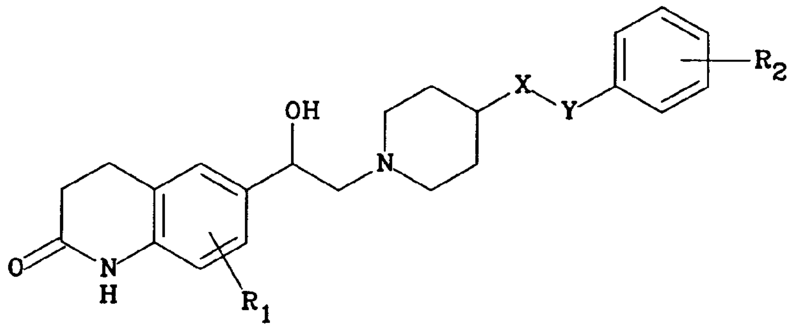

| N° | $R_1$ | $R_2$ | X | Y | Sel | F (°C) |
|---|---|---|---|---|---|---|
| 1 | H | 2-F | $CH_2$ | $CH_2$ | - | 161-162 |
| 2 | H | 3-F | $CH_2$ | $CH_2$ | - | 165-166 |
| 3 | H | 4-F | $CH_2$ | $CH_2$ | - | 159-160 |
| 4 | H | 4-Cl | $CH_2$ | $CH_2$ | - | 189-190 |
| 5 | H | 3-$CH_3$ | $CH_2$ | $CH_2$ | - | 163-164 |
| 6 | H | 4-$CH_3$ | $CH_2$ | $CH_2$ | - | 174-176 |
| 7 | H | 4-OH | $CH_2$ | $CH_2$ | - | 225-226 |
| 8 | H | 4-$OCH_3$ | $CH_2$ | $CH_2$ | - | 192-193 |
| 9 | H | 4-$CF_3$ | $CH_2$ | $CH_2$ | - | 163-164 |
| 9a | énantiomère lévogyre | | | | - | 172-173 |
| 9b | énantiomère dextrogyre | | | | - | 174-175 |
| 10 | H | H | $CH_2$ | $CH_2CH_2$ | - | 164-165 |
| 11 | H | H | $CH_2$ | CO | - | 186-187 |
| 12 | H | H | CO | $CH_2$ | - | 183-184 |
| 13 | H | 4-F | CO | $CH_2$ | - | 171-172 |

| N° | $R_1$ | $R_2$ | X | Y | Sel | F (°C) |
|---|---|---|---|---|---|---|
| 14 | H | 4-$CH_3$ | CO | $CH_2$ | - | 199-200 |
| 15 | 8-F | H | $CH_2$ | $CH_2$ | - | 138-140 |
| 16 | 8-F | 2-F | $CH_2$ | $CH_2$ | - | 139-140 |
| 17 | 8-F | 3-F | $CH_2$ | $CH_2$ | - | 116-117 |
| 18 | 8-F | 4-F | $CH_2$ | $CH_2$ | - | 163-164 |
| 19 | 8-F | 4-$OCH_3$ | $CH_2$ | $CH_2$ | - | 166-167 |
| 20 | 8-F | 4-$CF_3$ | $CH_2$ | $CH_2$ | - | 172-173 |
| 21 | 8-F | 4-Cl | $CH_2$ | $CH_2$ | fum. | 215-216 |
| 22 | H | 3-Cl | $CH_2$ | $CH_2$ | ox. | 219-220 |
| 23 | H | 4-F | $CH_2$ | CO | - | 192-193 |
| 24 | 8-F | 4-F | $CH_2$ | CO | - | 176-177 |
| 25 | 8-F | 2-$CH_3$ | $CH_2$ | $CH_2$ | - | 139-140 |
| 26 | 8-$CH_3$ | H | $CH_2$ | $CH_2$ | - | 195-196 |
| 27 | 8-$CH_3$ | 3-$CF_3$ | $CH_2$ | $CH_2$ | - | 164-165 |
| 28 | H | 2-$CF_3$ | $CH_2$ | $CH_2$ | - | 172-173 |
| 29 | 8-$CH_3$ | 2-F | $CH_2$ | $CH_2$ | - | 166-167 |
| 30 | H | 2-$CH_3$ | $CH_2$ | $CH_2$ | - | 161-162 |
| 31 | 8-$CH_3$ | 4-F | $CH_2$ | $CH_2$ | - | 180-181 |
| 32 | 8-$CH_3$ | 4-$CF_3$ | $CH_2$ | $CH_2$ | - | 190-191 |
| 33 | 8-$CH_3$ | 4-$CH_3$ | $CH_2$ | $CH_2$ | - | 206-207 |
| 34 | 8-F | 2-$CF_3$ | $CH_2$ | $CH_2$ | - | 123-124 |

| N° | $R_1$ | $R_2$ | X | Y | Sel | F (°C) |
|---|---|---|---|---|---|---|
| 35 | 8-$CH_3$ | 2-$CF_3$ | $CH_2$ | $CH_2$ | - | 172-173 |
| 36 | 8-$CH_3$ | 2-$CH_3$ | $CH_2$ | $CH_2$ | - | 185-186 |
| 37 | 8-$CH_3$ | 3-$CH_3$ | $CH_2$ | $CH_2$ | - | 186-187 |
| 38 | 8-$CH_3$ | 4-F | $CH_2$ | CO | - | 178-179 |
| 39 | 7-F | H | $CH_2$ | $CH_2$ | - | 199-200 |
| 40 | H | 3,5-$F_2$ | $CH_2$ | $CH_2$ | - | 173-174 |
| 41 | 8-F | 3,5-$F_2$ | $CH_2$ | $CH_2$ | - | 146-147 |
| 42 | 8-$CH_3$ | 3,5-$F_2$ | $CH_2$ | $CH_2$ | - | 162-163 |
| 43 | 7-F | 2-F | $CH_2$ | $CH_2$ | - | 198-199 |
| 44 | H | 2,4-$F_2$ | $CH_2$ | $CH_2$ | - | 171-172 |
| 45 | H | 3,4-$F_2$ | $CH_2$ | $CH_2$ | - | 163,5-164,5 |
| 46 | 7-F | 2-$CH_3$ | $CH_2$ | $CH_2$ | - | 199-200 |
| 47 | 8-$CH_3$ | 2,4-$F_2$ | $CH_2$ | $CH_2$ | - | 161-162 |
| 48 | 8-$CH_3$ | 3,4-$F_2$ | $CH_2$ | $CH_2$ | - | 156-157 |
| 49 | 8-F | 3,4-$F_2$ | $CH_2$ | $CH_2$ | - | 148-149 |
| 50 | 8-F | 2,4-$F_2$ | $CH_2$ | $CH_2$ | - | 164-165 |
| 51 | H | 4-$NHCOCH_3$ | $CH_2$ | $CH_2$ | - | 205-206 |
| 52 | H | 4-$CF_3$ | $CH_2$ | CO | - | 192-193 |
| 53 | H | 4-$NHSO_2CH_3$ | $CH_2$ | $CH_2$ | - | 208-209 |
| 54 | 8-F | H | $CH_2$ | $CH_2CH_2$ | - | 131-132 |
| 55 | 8-$CH_3$ | H | $CH_2$ | $CH_2CH_2$ | - | 152-153 |

Les composés de l'invention ont fait l'objet d'essais pharmacologiques qui ont mis en évidence leur intérêt comme substances actives de médicaments à visées neuroprotectrices.

Ainsi, en particulier, l'activité neuroprotectrice du composé de l'invention a été montrée dans un modèle d'ischémie focale par ligature de l'artère cérébrale moyenne chez la souris, selon une méthode analogue à celle décrite dans Brain Research, **522,** (1990), 290-307.
Six jours après occlusion de l'artère cérébrale moyenne par électrocoagulation sous anesthésie à l'halothane, les souris sont à nouveau anesthésiées et le cortex cérébral ispsilatéral à l'occlusion est prélevé. Après homogénéisation du tissu, l'étendue de l'infarctus cérébral est évaluée par mesure de l'augmentation de la densité des sites benzodiazépiniques périphériques ($\omega_3$) à l'aide du composé [$^3$H]PK 11195 de New England Nuclear.
Les traitements sont administrés curativement aux temps 5 minutes, 3 heures, 6 heures, 18 heures et 24 heures, par voie intrapéritonéale.
Certains composés de l'invention diminuent la densité des sites benzodiazépiniques périphériques d'environ 70% à la dose de 10 mg/kg.

Les composés de l'invention ont aussi fait l'objet d'un essai d'inhibition de la liaison du [$^3$H]ifenprodil aux récepteurs sensibles aux polyamines du cortex cérébral de rat, d'après le protocole décrit par Schoemaker et coll., *Eur. J. Pharmacol.,* **176,** 249-250, (1990).
Le rat mâle Sprague-Dawley de 150 à 230 g est sacrifié et le cortex cérébral est homogénéisé dans 20 volumes de tampon Tris-HCl à 50mM (pH=7,4 à 0°C) glacé, au moyen d'un appareil Ultra-Turrax™ (Ikawerk) ou Polytron™ (Kinematica).
L'homogénat est lavé deux fois par centrifugation pendant 10minutes à 45000xg, le culot étant remis en suspension dans du tampon frais. Le culot final est repris dans 20 volumes du même tampon.
Un aliquote de 100 µl de cette suspension est incubé dans un volume final de 1000 µl avec 1 nM de [$^3$H]ifenprodil (activité spécifique : 30 à 35 Ci/mmole) pendant 120 minutes à 0°C, en présence de 3 µM de GBR 12909 (Research Biochemicals Inc., Natick, MA, USA), en l'absence ou en présence de substance compétitrice.
Après incubation, le mélange est dilué avec 5 ml de tampon Tris-HCl à 50mM (pH=7,4 à 0°C) glacé et les membranes sont récupérées par filtration sur des filtres Whatman GF/B™ prétraités avec de la polyéthylèneimine à 0,05%, puis lavés avec deux fois 5 ml de tampon glacé.
On détermine la liaison non spécifique avec l'ifenprodil 10µM, on analyse les données selon les méthodes usuelles, et on calcule la concentration $CI_{50}$, concentration qui inhibe de 50% la liaison du [$^3$H]ifenprodil.
Les $CI_{50}$ varient de 2 nM à 10 µM ($2{\cdot}10^{-9}$ à $1{\cdot}10^{-5}$M).

Les composés de l'invention ont encore fait l'objet d'un essai d'inhibition de la liaison du [$^3$H]ifenprodil aux récepteurs sigma du cortex cérébral de rat (Schoemaker et coll., *Eur. J. Pharmacol.,* **183,** 1670, (1990)).
Le rat mâle Sprague-Dawley de 150 à 230 g est sacrifié et le cortex cérébral est homogénéisé dans 20 volumes de tampon Tris-HCl à 50mM (pH=7,4 à 25°C) glacé, au moyen d'un appareil Ultra-Turrax™ (Ikawerk) ou Polytron™ (Kinematica).
L'homogénat est lavé deux fois par centrifugation pendant 10 minutes à 45000xg, le culot étant remis en suspension dans du tampon frais. Le culot final est repris dans 20 volumes du même tampon.
Un aliquote de 100 µl de cette suspension est incubé dans un volume final de 1000 µl avec 0,5 nM de [$^3$H]ifenprodil (activité spécifique : 30 à 35 Ci/mmole) pendant 30 minutes à 37°C, en l'absence ou en présence de substance compétitrice. Après incubation les membranes sont récupérées par filtration sur des filtres Whatman GF/B™ prétraités avec de la polyéthylèneimine à 0,05%, puis lavés avec deux fois 5 ml de tampon glacé.
On détermine la liaison non spécifique avec de l'ifenprodil 10µM, on analyse les données selon les méthodes usuelles, et on calcule la concentration $CI_{50}$, concentration qui inhibe de 50% la liaison du [$^3$H]ifenprodil.
Les $CI_{50}$ des composés de l'invention varient de 2,5 à 800 nM ($2{,}5{\cdot}10^{-9}$ à $8{\cdot}10^{-7}$M).

Enfin les composés de l'invention ont été testés quant à leur activité vis-à-vis des convulsions maximales induites chez la souris par électrochoc supramaximal.
Le protocole de cet essai est décrit par E. A. Swinyard et J. H. Woodhead dans *Antiepileptic Drugs,* Raven Press, New York, 111-126 (1982).
30 minutes après administration intrapéritonéale du composé à tester, on note le nombre de souris présentant des convulsions (extensions des pattes arrière), immédiatement après application d'un courant électrique (0,4 s, 60 mA, 50 Hz) à l'aide d'électrodes transcornéennes. Les résultats sont exprimés par la $DA_{50}$, dose qui protège 50% des animaux, calculée selon la méthode de J. T. Lichtfield et F. Wilcoxon *(J. Pharm. Exp. Ther.,* **96,** 99-113 (1949)) à partir de 3 ou 4 doses administrées chacune à un groupe de 8 à 10 souris. Les $DA_{50}$ des composés les plus actifs dans cet essai sont de l'ordre de 10 mg/kg par la voie intrapéritonéale.

Les résultats des essais effectués sur les composés de l'invention suggèrent qu'ils peuvent être utilisés pour le traitement et la prévention de désordres cérébraux tels que ceux qui sont consécutifs, par exemple, à une attaque ischémique, un arrêt cardiaque ou respiratoire, une thrombose ou une embolie cérébrale, pour le traitement de la démence consécutive aux infarctus multiples, de la démence sénile, par exemple de la maladie

d'Alzheimer ou de la maladie de Pick, pour le traitement de l'atrophie olivo-ponto-cérébellaire et d'autres maladies neurodégénératives telles que la chorée de Huntington, pour le traitement de la schizophrénie, pour le traitement des traumatismes crâniens ou spinaux, pour le traitement des états convulsifs, comme antiémétiques lors du traitement de certains cancers avec le cisplatine, et pour le traitement du SIDA (voir *Science,* **250,** 1593 (1990)).

A cet effet il peut être présenté sous toutes formes pharmaceutiques adaptées à l'administration entérale ou parentérale, en association avec des excipients appropriés, par exemple sous forme de comprimés, dragées, gélules, capsules, suppositoires, solutions ou suspensions buvables ou injectables, dosés pour permettre une administration journalière de 1 à 1000 mg de substance active.

## Revendications

1. Composé, sous forme d'isomère optique pur ou de mélange de deux énantiomères, répondant à la formule générale (I)

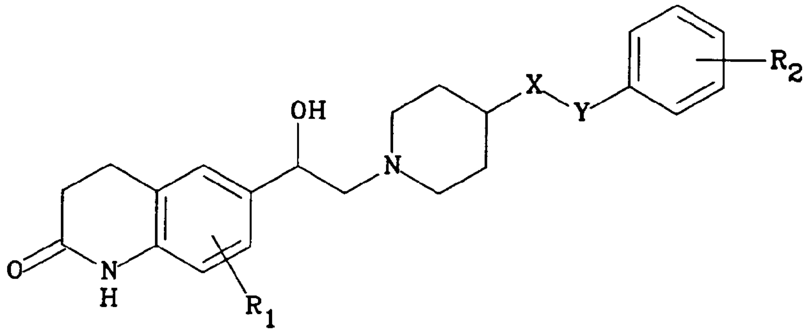

(I)

dans laquelle
$R_1$ représente un atome d'hydrogène ou d'halogène ou un groupe méthyle,
$R_2$ représente un ou deux substituants choisis parmi les atomes d'hydrogène et d'halogènes et les groupes $(C_{1-4})$alkyle, hydroxy, $(C_{1-4})$alcoxy, trifluorométhyle, acétylamino et méthylsulfonylamino, et
soit X représente un groupe $CH_2$ et Y représente un groupe $CH_2$, $(CH_2)_2$ ou CO,
soit X représente un groupe CO et Y représente un groupe $CH_2$, étant exclu le composé dans la formule duquel $R_1$ et $R_2$ représentent chacun un atome d'hydrogène et X et Y représentent chacun un groupe $CH_2$,
à l'état de base libre ou de sel d'addition à un acide pharmaceutiquement acceptable.

2. Composés selon la revendication 1, en l'espèce la 6-[1-hydroxy-2-[4-[2-[4-(trifluorométhyl)phényl]éthyl]pipéridin-1-yl]éthyl]-3,4-dihydroquinoléin-2(1*H*)-one, et ses énantiomères lévogyre et dextrogyre.

3. Procédé de préparation d'un composé selon la revendication 1, caractérisé en ce qu'on fait réagir un composé de formule générale (II)

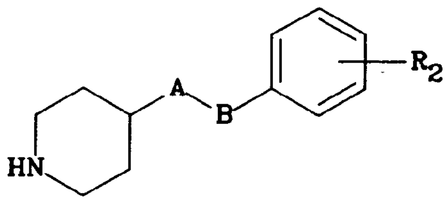

(II)

dans laquelle
soit A est un groupe $CH_2$ et B est un groupe $CH_2$ ou $(CH_2)_2$
soit A ou B est un groupe $CH_2$ et l'autre est un groupe 1,3-dioxolan-2-yle,
et $R_2$ est tel que défini dans la revendication 1,
avec un composé de formule générale (III)

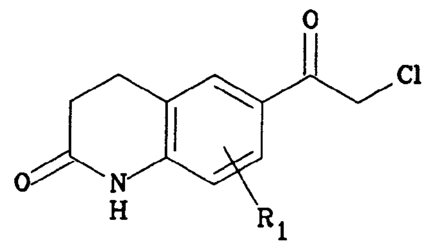

(III)

dans laquelle $R_1$ est tel que défini dans la revendication 1, pour obtenir un composé de formule générale (IV)

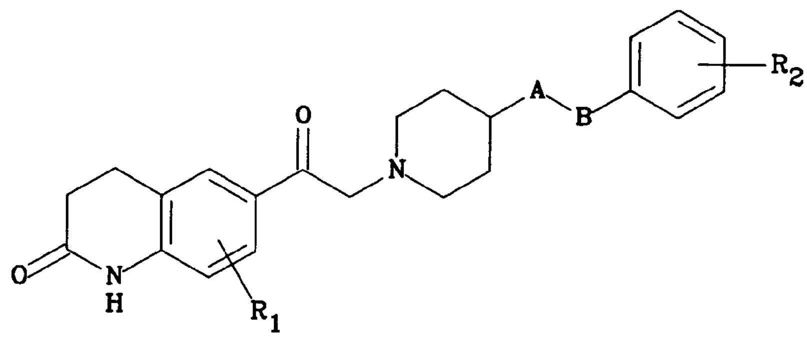

(IV)

puis on réduit la fonction cétone en fonction alcool et, si nécessaire, on hydrolyse le groupe 1,3-dioxolan-2,2-diyle en groupe CO.

4. Médicament caractérisé en ce qu'il consiste en un composé selon l'une des revendications 1 et 2.

5. Composition pharmaceutique, caractérisée en ce qu'elle contient un composé selon l'une des revendications 1 et 2, associé à un excipient.

**Revendications pour les Etats contractants suivants : ES, GR**

1. Procédé de préparation d'un composé répondant à la formule générale (I)

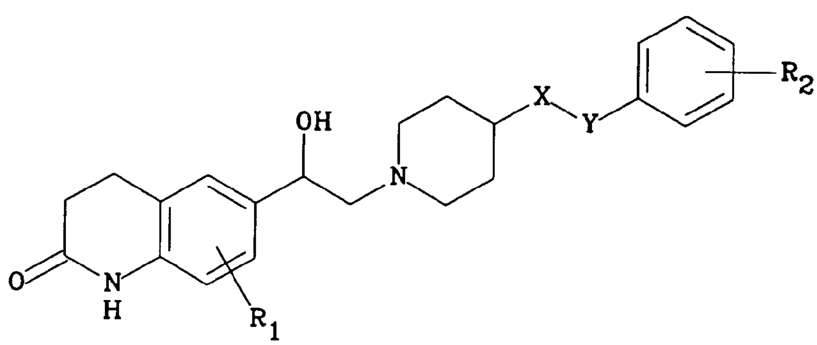

(I)

dans laquelle
$R_1$ représente un atome d'hydrogène ou d'halogène ou un groupe méthyle,
$R_2$ représente un ou deux substituants choisis parmi les atomes d'hydrogène et d'halogènes et les groupes $(C_{1-4})$alkyle, hydroxy, $(C_{1-4})$alcoxy, trifluorométhyle, acétylamino et méthylsulfonylamino, et
soit X représente un groupe $CH_2$ et Y représente un groupe $CH_2$, $(CH_2)_2$ ou CO,
soit X représente un groupe CO et Y représente un groupe $CH_2$, étant exclu le composé dans la formule duquel $R_1$ et $R_2$ représentent chacun un atome d'hydrogène et X et Y représentent chacun un groupe $CH_2$,

procédé caractérisé en ce qu'on fait réagir un composé de formule générale (II)

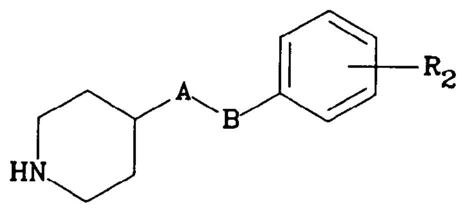

(II)

dans laquelle
soit A est un groupe $CH_2$ et B est un groupe $CH_2$ ou $(CH_2)_2$
soit A ou B est un groupe $CH_2$ et l'autre est un groupe 1,3-dioxolan-2-yle,
et $R_2$ est tel que défini ci-dessus,
avec un composé de formule générale (III)

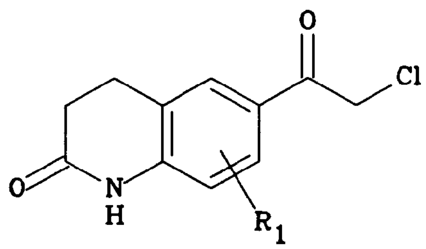

(III)

dans laquelle $R_1$ est tel que défini ci-dessus, pour obtenir un composé de formule générale (IV)

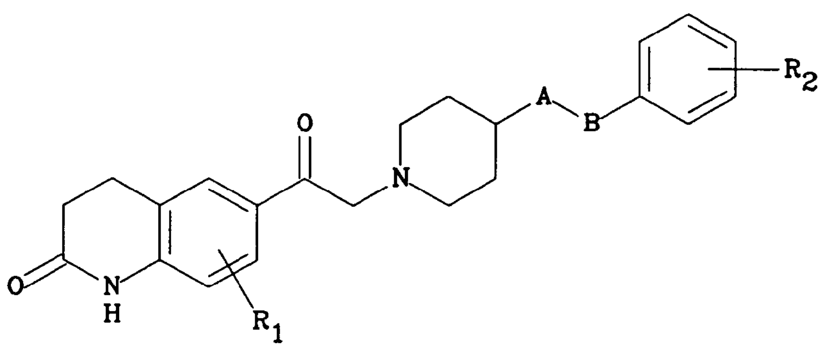

(IV)

puis on réduit la fonction cétone en fonction alcool et, si nécessaire, on hydrolyse le groupe 1,3-dioxolan-2,2-diyle en groupe CO.

Office européen des brevets

# RAPPORT DE RECHERCHE EUROPEENNE

Numero de la demande

EP 92 40 1769

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 389 352 (ADIR ET COMPAGNIE)<br>* page 3 - page 4, ligne 41 *<br>--- | 1,4 | C07D401/06<br>A61K31/45 |
| Y | EP-A-0 351 282 (SYNTHELABO)<br>* page 2 *<br>* page 14, ligne 62 - page 15, ligne 33 *<br>--- | 1,4 | |
| Y | EP-A-0 409 236 (EISAI CO.)<br>* le document en entier *<br>--- | 1,4 | |
| Y | EP-A-0 378 207 (TAKEDA CHEMICAL INDUSTRIES LTD.)<br>* page 38, ligne 20 - page 39, ligne 19; exemples 43,44,45 *<br>* page 2 - ligne 50 *<br>--- | 1,4 | |
| Y | EP-A-0 279 681 (YAMANOUCHI PHARMACEUTICAL CO.LTD)<br>* page 38, ligne 45 - page 39; exemples 29,30,31,32 *<br>--- | 1,4 | **DOMAINES TECHNIQUES RECHERCHES (Int. Cl.5)** |
| P,D,A | EP-A-0 481 853 (SYNTHELABO)<br>* le document en entier *<br>----- | 1,4 | C07D |

Le présent rapport a été établi pour toutes les revendications

| Lieu de la recherche | Date d'achèvement de la recherche | Examinateur |
|---|---|---|
| BERLIN | 14 OCTOBRE 1992 | KYRIAKAKOU G. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)